(19) Europäisches Patentamt
European Patent Office
Office européen des brevets

(11) EP 2 346 950 B1

(12) **EUROPÄISCHE PATENTSCHRIFT**

(45) Veröffentlichungstag und Bekanntmachung des Hinweises auf die Patenterteilung:
**31.07.2013 Patentblatt 2013/31**

(21) Anmeldenummer: **10742765.0**

(22) Anmeldetag: **09.08.2010**

(51) Int Cl.:
***C09C 1/00*** *(2006.01)*

(86) Internationale Anmeldenummer:
**PCT/EP2010/004867**

(87) Internationale Veröffentlichungsnummer:
**WO 2011/020572 (24.02.2011 Gazette 2011/08)**

# (54) HOCHGLÄNZENDE MEHRSCHICHTPERLGLANZPIGMENTE MIT SILBERNER INTERFERENZFARBE UND ENGER GRÖSSENVERTEILUNG UND VERFAHREN ZU DEREN HERSTELLUNG

HIGH-GLOSS MULTILAYER EFFECT PIGMENTS HAVING A SILVER INTERFERENCE COLOR AND A NARROW SIZE DISTRIBUTION, AND METHOD FOR THE PRODUCTION THEREOF

PIGMENTS NACRÉS MULTICOUCHE TRÈS BRILLANTS DOTÉS D'UNE TEINTE DE POLARISATION ARGENTÉE ET D'UNE ÉTROITE DISTRIBUTION GRANULOMÉTRIQUE ET LEUR PROCÉDÉ DE PRODUCTION

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **19.08.2009 DE 102009037935**

(43) Veröffentlichungstag der Anmeldung:
**27.07.2011 Patentblatt 2011/30**

(73) Patentinhaber: **Eckart GmbH**
**91235 Hartenstein (DE)**

(72) Erfinder:
- **SCHUMACHER, Dirk**
  **91257 Pegnitz (DE)**
- **GRÜNER, Michael**
  **91275 Auerbach (DE)**
- **KAUPP, Günter**
  **91284 Neuhaus (DE)**

(74) Vertreter: **Louis Pöhlau Lohrentz**
**Patentanwälte**
**Postfach 30 55**
**90014 Nürnberg (DE)**

(56) Entgegenhaltungen:
**WO-A1-2009/103322**
**WO-A2-03/006558**
**WO-A2-2006/110359**
**US-A1- 2006 042 509**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 346 950 B1

## Beschreibung

Die vorliegende Erfindung betrifft hochglänzende Mehrschichtperlglanzpigmente mit silberner Interferenzfarbe, ein Verfahren zu deren Herstellung sowie deren Verwendung in kosmetischen Formulierungen, Kunststoffen, Folien, Textilien, keramischen Materialien, Gläsern, Beschichtungszusammensetzungen wie Farben, Druckfarben, Tinten, Lacken oder Pulverlacken.

Perlglanzpigmente mit silberner Interferenzfarbe werden gewöhnlich durch Beschichtung transparenter, niedrigbrechender Substrate mit einer dünnen $TiO_2$-Schicht hergestellt.

Silberfarbene Glanzpigmente auf der Basis von mehrfach beschichteten plättchenförmigen Substraten sind aus EP 1 213 330 A1 bekannt. Zwischen zwei hochbrechenden $TiO_2$-Schichten mit einer Schichtdicke von 5 bis 200 nm wird hier eine niedrigbrechende Schicht mit einer Schichtdicke von 10 bis 300 nm auf dem zu beschichtenden Substrat aufgebracht. Optional kann die äußere hochbrechende Schicht zur Erhöhung der Licht-, Temperatur- und Wetterstabilität mit einer Schutzschicht versehen werden. Den Beispielen ist zu entnehmen, dass der Glanz der erfindungsgemäßen Beispiele im Vergleich zu einem kommerziell erhältlichen Perlglanzpigment um 1,3 bis 2,9 Glanzeinheiten ansteigt.

Goniochromatische Glanzpigmente werden in EP 0 753 545 B2 beschrieben. Mindestens ein Schichtpaket aus einer farblosen niedrigbrechenden und einer reflektierenden, selektiv oder nichtselektiv absorbierenden Beschichtung sowie optional einer äußeren Schutzschicht wird hier auf ein mehrfach beschichtetes, hochbrechendes, nichtmetallisches, plättchenförmiges Substrat aufgebracht. Die Schichtdicke der farblosen niedrigbrechenden Beschichtung verringert sich bei steigender Anzahl der auf dem Substrat aufgebrachten Schichtpakete. Die goniochromatischen Glanzpigmente zeigen einen winkelabhängigen Farbwechsel zwischen mehreren intensiven Interferenzfarben.

Gemäß WO 2004/067645 A2 wird ein transparentes Substrat mit einer ungeraden Anzahl, mindestens drei, abwechselnd hoch- und niedrigbrechenden Schichten beschichtet. Der Brechungsindexunterschied der aneinandergrenzenden Schichten liegt bei mindestens 0,2. Mindestens eine der Schichten unterscheidet sich in ihrer optischen Dicke von den anderen. Somit besitzen die resultierenden Mehrschichteffektpigmente keinen Schichtaufbau, bei dem die optische Dicke jeder Schicht ein ungeradzahliges Vielfaches eines Viertels der zu interferierenden Lichtwellenlänge beträgt (kein "quarter-wave-stack"-Aufbau).

In WO 2006/088759 A1 werden die Mehrschichteffektpigmente mit Titandioxid, einer niedrigbrechenden Schicht mit einer optischen Schichtdicke von mindestens 150 nm und anschließend wieder mit einer hochbrechenden Schicht umfassend Titandioxid mit einer optischen Schichtdicke von ca. 45 bis 240 nm beschichtet. Die erste Titandioxidschicht verleiht dem Substrat einen silbernen Glanz, während die resultierenden Mehrschichteffektpigmente jedoch keinen silbernen Glanz besitzen. Aufgrund der optischen Schichtdicke der niedrigbrechenden Schicht besitzen die Mehrschichteffektpigmente einen Farbflop. Analog zu WO 2004/067645 A2 weisen auch hier die aneinandergrenzenden Schichten einen Brechungsindexunterschied von mindestens 0,2 auf. Ebenfalls soll kein Schichtaufbau, bei dem die optische Dicke jeder Schicht ein ungeradzahliges Vielfaches eines Viertels der zu interferierenden Lichtwellenlänge beträgt (kein "quarter-wave-stack"-Aufbau) gegeben sein.

US 2006/0042509 A beschreibt mit $SiO_2$ und Ceroxid beschichtete Perlglanzpigmente.

WO 2009/103322 A1 beschreibt Effektpigmente basierend auf künstlich hergestellten Substraten mit enger Größenverteilung.

Mehrschichtige Interferenzpigmente mit kräftigen Interferenzfarben und/ oder einer starken Winkelabhängigkeit der Interferenzfarben, bestehend aus einem transparenten Trägermaterial, das mit alternierenden Schichten von Metalloxiden mit niedriger und hoher Brechzahl beschichtet ist, werden in EP 0 948 572 B1 beschrieben. Die Differenz der Brechzahlen beträgt mindestens 0,1. Die Anzahl und Dicke der Schichten ist abhängig vom gewünschten Effekt und dem verwendeten Substrat. Betrachtet man den Aufbau $TiO_2$-$SiO_2$-$TiO_2$ auf einem Glimmersubstrat, so lassen sich bei Verwendung optisch dünner $TiO_2$- und $SiO_2$-Schichten mit einer Schichtdicke < 100 nm beispielsweise Pigmente mit blauer Interferenzfarbe erhalten, die farbkräftiger als reine $TiO_2$-Glimmerpigmente sind. Durch die Auffällung dicker $SiO_2$-Schichten mit einer Schichtdicke > 100 nm werden Pigmente mit einer stark ausgeprägten Winkelabhängigkeit der Interferenzfarbe erhalten.

JP 07246366 beschreibt ein optisches Interferenzmaterial, das aus alternierenden hochbrechenden und niedrigbrechenden Schichten aufgebaut ist, wobei die optische Dicke jeder Schicht ein ungeradzahliges Vielfaches eines Viertels der zu interferierenden Lichtwellenlänge beträgt ("quater-wave-stack"-Aufbau).

Interferenzpigmente auf der Basis von mehrfach beschichteten plättchenförmigen Substraten, die mindestens eine Schichtenfolge aus einer hochbrechenden, einer farblosen niedrigbrechenden, einer nicht absorbierenden hochbrechenden und gegebenenfalls einer äußeren Schutzschicht aufweisen, können nach EP 1 025 168 B1 hergestellt werden. Zwischen dem Substrat und der ersten Schicht bzw. zwischen den einzelnen Schichten können sich weitere farbige oder farblose Metalloxidschichten befinden. Die Interferenzpigmente können mehrere, gleiche oder verschiedene Kombinationen an Schichtpaketen enthalten, bevorzugt ist aber die Belegung des Substrats mit nur einem Schichtpaket. Zur Intensivierung des Farbflops können die Interferenzpigmente bis zu vier Schichtpakete enthalten, wobei die Dicke aller Schichten auf dem Substrat 3 μm nicht überschreiten sollte.

Mehrschichtpigmente basierend auf Glasflakes, die mit mindestens drei alternierenden Schichten mit hohem und niedrigem Brechungsindex beschichtet sind, werden in WO 2003/006558 A2 beschrieben. Die Glasflakes besitzen hier eine Dicke von < 1μm. Die Mehrschichtpigmente weisen neben intensiven Farben einen starken Farbflop auf.

In WO 2004/055119 A1 werden Interferenzpigmente auf der Basis von beschichteten plättchenförmigen Substraten beschrieben. Die Substrate sind hierbei mit einer ersten Schicht aus $SiO_2$ belegt, auf die anschließend eine hochbrechende Schicht, bestehend aus beispielsweise $TiO_2$, $ZrO_2$, $SnO_2$, $Cr_2O_3$, $Fe_2O_3$ oder $Fe_3O_4$ bzw. ein Interferenzsystem aus alternierenden hoch- und niedrigbrechenden Schichten aufgebracht ist. Optional können die Pigmente noch eine äußere Schutzschicht aufweisen. Auf diese Weise werden silberweiße Interferenzpigmente bzw. Interferenzpigmente mit brillanten Interferenzfarben erhalten, die sich durch anwendungstechnische Eigenschaften, wie mechanische Stabilität und Photostabilität auszeichnen, allerdings keinen hohen Glanz aufweisen. Die Interferenzpigmente zeigen keine oder nur eine geringe Winkelabhängigkeit der Farbe.

Thermisch und mechanisch stabile Effektpigmente basierend auf dünnen Glasplättchen mit einer Dicke ≤ 1,0 μm sind aus WO 2002/090448 A2 bekannt. Die Effektpigmente können mit einer oder mehreren hoch- und/ oder niedrigbrechenden Schicht(en) belegt sein. Die Glasflakes besitzen eine Erweichungstemperatur von ≥ 800°C.

Die optischen Eigenschaften von Effektpigmenten können nach WO 2006/110359 A2 durch eine geeignete Partikelgrößenverteilung beeinflusst werden. Die hier beschriebenen klassierten mit einer einzigen Metalloxidschicht beschichteten Glasplättchen weisen einen $D_{10}$ von wenigstens 9,5 μm, vorzugsweise von 9,5 μm, auf. Nachteilig ist, dass die Pigmente einen Größenbereich mit einem $D_{90}$-Wert von maximal 85 μm, vorzugsweise von etwa 45 μm, aufzuweisen haben.

Der vorliegenden Erfindung liegt die Aufgabe zugrunde, hochglänzende Mehrschichtperlglanzpigmente mit silberner Interferenzfarbe bereitzustellen, die den für Perlglanzpigmente typischen Tiefenglanz bei gleichzeitiger Transparenz aufweisen und die gegenüber den aus dem Stand der Technik bekannten Mehrschichtperlglanzpigmenten einen erhöhten Glanz besitzen. Weiterhin ist es Aufgabe der Erfindung, ein Verfahren zur Herstellung dieser Mehrschichtperlglanzpigmente zur Verfügung zu stellen.

Die der Erfindung zugrundeliegende Aufgabe wurde durch Bereitstellung von Mehrschichtperlglanzpigmenten mit silberner Interferenzfarbe basierend auf plättchenförmigen transparenten Substraten mit optisch wirksamer Beschichtung gelöst, wobei die optisch wirksame Beschichtung wenigstens

a) eine hochbrechende Schicht A mit einem Brechungsindex n ≥ 1,8
b) eine niedrigbrechende Schicht B mit einem Brechungsindex n < 1,8
c) eine hochbrechende Schicht C mit einem Brechungsindex n ≥ 1,8 sowie
d) optional eine äußere Schutzschicht D

umfasst und die Mehrschichtperlglanzpigmente die Kennzahlen $D_{10}$, $D_{50}$, $D_{90}$ aus der Summenhäufigkeitsverteilung der volumengemittelten Größenverteilungsfunktion mit einem Span $\Delta D$ von 0,7 - 1,4 aufweisen. Der Span $\Delta D$ wird gemäß der Formel $\Delta D = (D_{90} - D_{10})/ D_{50}$ berechnet.

Bevorzugte Weiterbildungen sind in den abhängigen Ansprüchen 2 bis 10 angegeben.

Die der Erfindung zugrundeliegende Aufgabe wurde weiterhin gelöst durch Bereitstellung eines Verfahrens zur Herstellung der erfindungsgemäßen Mehrschichtperlglanzpigmente, welches folgende Schritte umfasst:

i) Größenklassieren der zu beschichtenden plättchenförmigen transparenten Substrate
ii) Beschichten der plättchenförmigen transparenten Substrate mit wenigstens oben genannten Schichten A bis C sowie optional Schicht D.

Alternativ können die plättchenförmigen transparenten Substrate zuerst mit wenigstens oben genannten Schichten A bis C sowie optional Schicht D beschichtet werden und die so erhaltenen Mehrschichtperlglanzpigmente mit silberner Interferenzfarbe anschließend nach Größe klassiert werden.

Vorzugsweise erfolgt das Beschichten der plättchenförmigen transparenten Substrate in Schritt ii) nach dem Größenklassieren in Schritt i).

Des Weiteren ist Gegenstand der Erfindung die Verwendung der erfindungsgemäßen Mehrschichtperlglanzpigmente in kosmetischen Formulierungen, Kunststoffen, Folien, Textilien, keramischen Materialien, Gläsern und Beschichtungszusammensetzungen, wie Farben, Druckfarben, Tinten, Lacken und Pulverlacken. Gegenstand der Erfindung sind mithin Zubereitungen, die die erfindungsgemäßen Mehrschichtperlglanzpigmente enthalten. Die Erfindung ist auch auf Gegenstände gerichtet, die mit den erfindungsgemäßen Mehrschichtperlglanzpigmenten versehen, beispielsweise lackiert oder bedruckt, sind. Somit sind lackierte Gegenstände, wie Karosserien, Fassadenelemente, etc. oder bedruckte Gegenstände, wie Papier, Pappe, Folien, Textilien, etc., ebenfalls Bestandteil der vorliegenden Erfindung.

Die Empfindung einer Farbe als matt, blass oder kräftig hängt maßgeblich von ihrer Farbsättigung, dem sogenannten Chroma oder der Buntheit ab. Dabei wird das Chroma durch die enthaltene Menge an Grau bestimmt. Je höher der Graugehalt, desto geringer ist die Farbsättigung.

Betrachtet man einen Punkt F im CIELab-Farbsystem, so ist dieser über die drei Koordinaten L* (Helligkeit), a* (rot-grün-Achse) und b* (gelb-blau-Achse) definiert. Die Farbkoordinaten a* und b* können auch über Polarkoordinaten C* (Chroma) und h* (Farbwinkel, Farbort) ausgedrückt werden, wobei die Definition wie folgt gegeben ist:

$$C^* = \sqrt{a^{*2} + b^{*2}}$$

$$h^* = \frac{180}{\pi} \cdot \arctan\left(\frac{b^*}{a^*}\right)$$

Das Chroma entspricht also der Länge des Vektors, der vom Ursprung des Koordinatensystems auf den zu definierenden Punkt F zeigt. Je geringer der C*-Wert ausfällt, desto näher liegt der Punkt F am unbunten, grauen Bereich des Farbkoordinatensystems. Das Chroma ist also der Abstand von der L*- oder Grau-Achse, die senkrecht auf der a*, b*-Ebene steht (Abbildung 1).

Effektpigmente, welche eine silberne Interferenzfarbe aufweisen, zeichnen sich durch geringe Chromawerte aus, d.h. es handelt sich um unbunte Interferenzfarben. Dies lässt sich auch anhand farbmetrischer Daten zeigen. Es wurden jeweils Rakelabzüge von Glasflakes, die mit einer (Zahl: 1) $TiO_2$-Schicht belegt sind, mit silberner Interferenzfarbe (MIRAGE Glamour Silver, Fa. Eckart), blauer Interferenzfarbe (MIRAGE Glamour Blue, Fa. Eckart) und roter Interferenzfarbe (MIRAGE Glamour Red, Fa. Eckart) in einem Nitrocelluloselack, der 6 Gew.-% an Pigmenten enthält, wobei sich die Gew.-% Angabe auf das Gesamtgewicht des Lackes bezieht, in einer Naßfilmdicke von 76 μm auf BYK-Gardner Schwarz-Weiß-Rakelkarte (Byko-Chart 2853) hergestellt und nachfolgend bei Raumtemperatur getrocknet. An diesen Rakelkarten wurden dann mit einem BYK-MAC (Fa. BYK Gardner) farbmetrische Auswertungen vorgenommen.

Betrachtet man die farbmetrischen Daten dieser drei ausgewählten Pigmente, so sieht man, dass das silberne Interferenzpigment bei einem Winkel von 15° außerhalb des Glanzes mit einem $C^*_{15}$-Wert von 6,0 den geringsten Chromawert aufweist.

Tabelle 1: Farbmetrische Daten von Glasflakes, die mit einer $TiO_2$-Schicht belegt sind

| Pigment | Winkel | L* | a* | b* | $C^*_{15}$ | h* |
|---|---|---|---|---|---|---|
| MIRAGE Glamour Blue | 15° | 46,3 | 1,5 | -22,9 | 22,9 | 273,7 |
| MIRAGE Glamour Red | 15° | 56,9 | 25,6 | -9,1 | 27,1 | 340,4 |
| MIRAGE Glamour Silver | 15° | 61,4 | -0,7 | -5,9 | 6,0 | 263,5 |

Die silberfarbene Interferenz kann man auch anhand einer Remissionskurve erkennen (Abbildung 2). Hierbei zeigen Effektpigmente mit silberner Interferenzfarbe eine weitgehend einheitlich hohe Reflexion im gesamten sichtbaren Wellenlängenbereich. Dagegen zeigen farbige Interferenzpigmente im sichtbaren Wellenlängenbereich Minima bzw. Maxima. So findet man für MIRAGE Glamour Red ein ausgeprägtes Maximum bei ca. 430 nm und 630 nm, während man bei ca. 510 nm ein ausgeprägtes Minimum detektieren kann. Dieses Remissionsverhalten führt zu einer hochchromatischen blaustichigen roten Interferenzfarbe. Dagegen zeigt MIRAGE Glamour Blue sein Maximum bei ca. 430 nm, aber ein Minimum bei ca. 600 nm, was zu einer blauen Interferenzfarbe führt. Das Perlglanzpigment MIRAGE Glamour Silver hingegen zeigt von ca. 400 nm bis 700 nm eine ausgeglichen hohe Reflexion, so dass hierbei kein Farbeindruck entsteht, sondern eine silberne Interferenzfarbe wahrgenommen wird.

Die EP 1 213 330 A1 offenbart Mehrschichtperlglanzpigmente mit silberner Interferenzfarbe. Gegenüber einschichtigen Perlglanzpigmenten wurde ein geringfügig verbesserter Glanz erhalten.

Die Erfinder haben überraschend festgestellt, dass die erfindungsgemäßen Mehrschichtperlglanzpigmente mit einem Span ΔD im Bereich von 0,7 bis 1,4 einen außerordentlich starken Glanz aufweisen.

Zur Charakterisierung der Größenverteilung der Mehrschichtperlglanzpigmente wird erfindungsgemäß der Span ΔD, definiert als $\Delta D = (D_{90} - D_{10})/D_{50}$, verwendet. Je kleiner der Span ist, desto enger ist die Größenverteilung.

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der Mehrschichtperlglanzpigmente einen Durchmesser aufweisen, der gleich oder kleiner als der jeweils angegebene Wert ist. Hierbei wird die Größenverteilungskurve mit einem Gerät der Fa. Malvern (Gerät: MALVERN Mastersizer 2000) gemäß Herstellerangaben bestimmt. Die Auswertung der Streulichtsignale erfolgte dabei nach der Mie-Theorie, welche auch Brechungs- und Absorptionsverhalten der Partikel beinhaltet (Abbildung 3).

Die erfindungsgemäßen Mehrschichtperlglanzpigmente besitzen einen Span $\Delta D$ in einem Bereich von 0,7 bis 1,4, vorzugsweise in einem Bereich von 0,7 bis 1,3, weiterhin bevorzugt in einem Bereich von 0,8 bis 1,2, besonders bevorzugt in einem Bereich von 0,8 bis 1,1. Bei weiterhin bevorzugten Ausführungsformen liegt der Span $\Delta D$ in einem Bereich von 0,85 bis 1,05.

Weisen die Mehrschichtperlglanzpigmente einen Span $\Delta D$ oberhalb von 1,4 auf, so werden keine hochglänzenden Mehrschichtperlglanzpigmente erhalten. Mehrschichtperlglanzpigmente unterhalb eines Spans $\Delta D$ von 0,7 können im Rahmen der üblichen Methoden nur sehr aufwändig und damit nicht mehr wirtschaftlich hergestellt werden.

Der Span $\Delta D$ des zu beschichtenden, plättchenförmigen transparenten Substrats entspricht im Wesentlichen dem des erfindungsgemäßen Mehrschichtperlglanzpigments und liegt bei $\leq 1,4$, vorzugsweise $\leq 1,3$, weiter bevorzugt $\leq 1,2$, besonders bevorzugt $\leq 1,1$ und ganz besonders bevorzugt bei $\leq 1,05$.

Die erfindungsgemäßen Mehrschichtperlglanzpigmente können eine beliebige mittlere Teilchengröße ($D_{50}$) aufweisen. Die $D_{50}$-Werte der erfindungsgemäßen Mehrschichtperlglanzpigmente liegen vorzugsweise in einem Bereich von 3 bis 350 $\mu$m. Bevorzugt weisen die erfindungsgemäßen Mehrschichtperlglanzpigmente einen $D_{50}$-Wert aus einem Bereich von 3 bis 15 $\mu$m oder aus einem Bereich von 10 bis 35 $\mu$m oder aus einem Bereich von 25 bis 45 $\mu$m oder aus einem Bereich von 30 bis 65 $\mu$m oder aus einem Bereich von 40 bis 140 $\mu$m oder aus einem Bereich von 135 bis 250 $\mu$m auf.

Die $D_{10}$-Werte der erfindungsgemäßen Mehrschichtperlglanzpigmente umfassen vorzugsweise einen Bereich von 1 bis 120 $\mu$m. Bevorzugt weisen die erfindungsgemäßen Mehrschichtperlglanzpigmente die in Tabelle 2 angegebenen Kombinationen von $D_{10}$-, $D_{50}$- und $D_{90}$-Werten auf. Hierbei werden die $D_{10}$-, $D_{50}$- und $D_{90}$-Werte der Tabelle 2 nur in der Art kombiniert, dass sich ein Span $\Delta D$ aus einem Bereich von 0,7 bis 1,4, vorzugsweise aus einem Bereich von 0,7 bis 1,3, weiterhin bevorzugt aus einem Bereich von 0,8 bis 1,2, besonders bevorzugt aus einem Bereich von 0,8 bis 1,1 und ganz besonders bevorzugt aus einem Bereich von 0,85 bis 1,05 ergibt. Kombinationen von $D_{10}$-, $D_{50}$- und $D_{90}$-Werten, die zu einem Span $\Delta D$ führen, der nicht im Bereich von 0,7 bis 1,4 liegt, sind keine erfindungsgemäßen Ausführungsformen.

Tabelle 2: Bevorzugte Kombinationen von Bereichen der $D_{10}$-, $D_{50}$- und $D_{90}$-Werte

| $D_{10}$($\mu$m) | $D_{50}$ ($\mu$m) | $D_{90}$ ($\mu$m) |
|---|---|---|
| 1 - 5 | 3 - 15 | 8 - 25 |
| 5 - 25 | 10 - 35 | 20 - 45 |
| 10 - 30 | 25 - 45 | 40 - 70 |
| 20 - 45 | 30 - 65 | 70 - 110 |
| 25 - 65 | 40 - 140 | 120 - 180 |
| 75 - 110 | 135 - 250 | 400 - 490 |

Dabei hat sich überraschenderweise gezeigt, dass die Größe der Mehrschichtperlglanzpigmente, charakterisiert durch den $D_{50}$- Wert, nicht entscheidend ist, sondern dass der Span $\Delta D = (D_{90} - D_{10})/ D_{50}$ in einem engen Bereich von 0,7 bis 1,4 liegt. Dabei können beispielsweise die $D_{50}$-Werte der Mehrschichtperlglanzpigmente bei 15, 20, 25, 30 $\mu$m oder auch bei 50, 80, 100, 150, 200, 250, 300 oder 350 $\mu$m liegen.

Geeignete zu beschichtende plättchenförmige transparente Substrate sind nichtmetallische, natürliche oder synthetische plättchenförmige Substrate. Die Substrate sind vorzugsweise im Wesentlichen transparent, bevorzugt transparent, d.h. für sichtbares Licht sind sie zumindest teilweise durchlässig.

Gemäß einer bevorzugten Ausführungsform der Erfindung können die plättchenförmigen transparenten Substrate aus der Gruppe, bestehend aus natürlichem Glimmer, synthetischem Glimmer, Glasflakes, $SiO_2$-Plättchen, $Al_2O_3$-Plättchen, Polymerplättchen, plättchenförmigem Bismuthoxychlorid, plättchenförmigen Substraten umfassend eine anorganisch-organische Mischschicht und deren Gemische, ausgewählt werden. Bevorzugt werden die plättchenförmigen transparenten Substrate aus der Gruppe, bestehend aus natürlichem Glimmer, synthetischem Glimmer, Glasflakes, $SiO_2$-Plättchen, $Al_2O_3$-Plättchen und deren Gemische, ausgewählt. Besonders bevorzugt werden die plättchenförmigen transparenten Substrate aus der Gruppe, bestehend aus natürlichem Glimmer, synthetischem Glimmer, Glasflakes und deren Gemische, ausgewählt. Ganz besonders bevorzugt sind Glasflakes und synthetischer Glimmer und deren Gemische.

Natürlicher Glimmer besitzt im Unterschied zu synthetischen plättchenförmigen transparenten Substraten den Nachteil, dass Verunreinigungen durch eingelagerte Fremdionen den Farbton verändern können, und dass die Oberfläche nicht ideal glatt ist, sondern Unregelmäßigkeiten, wie z.B. Stufen aufweisen kann.

Jedoch auch bei Verwendung von natürlichem Substrat hat sich überraschenderweise gezeigt, dass der Glanz einer Mehrzahl von Mehrschichtperlganzpigmenten gesteigert werden kann, wenn der Span $\Delta D$ in einem Bereich von 0,7 bis 1,4 liegt, verglichen mit einer Mehrzahl an herkömmlichen Mehrschichtperlglanzpigmenten mit breitem Span.

Synthetische Substrate wie beispielsweise Glasflakes oder synthetischer Glimmer dagegen weisen glatte Oberflächen, eine einheitliche Dicke innerhalb eines einzelnen Substratteilchens sowie scharfe Kanten auf. Somit bietet die Oberfläche nur wenig Streuzentren für einfallendes bzw. reflektiertes Licht und ermöglicht so nach Beschichtung höher glänzende Mehrschichtperlglanzpigmente als mit natürlichem Glimmer als Substrat. Als Glasflakes werden bevorzugt jene verwendet, die nach den in EP 0 289 240 A1, WO 2004/056716 A1 und der WO 2005/063637 A1 beschriebenen Verfahren hergestellt werden. Die als Substrat verwendbaren Glasflakes können beispielsweise eine Zusammensetzung entsprechend der Lehre der EP 1 980 594 B1 aufweisen.

Die mittlere geometrische Dicke der zu beschichtenden plättchenförmigen transparenten Substrate liegt in einem Bereich von 50 nm bis 5000 nm, bevorzugt in einem Bereich von 60 nm bis 3000 nm und besonders bevorzugt in einem Bereich von 70 nm bis 2000 nm. Bei einer Ausführungsform liegt die mittlere geometrische Dicke für Glasflakes als zu beschichtendes Substrat in einem Bereich von 750 nm bis 1500 nm. Derartige Glasflakes sind kommerziell auf breiter Basis verfügbar. Weitere Vorteile bieten dünnere Glasflakes. Dünnere Substrate führen zu einer geringeren Gesamtschichtdicke der erfindungsgemäßen Mehrschichtperlglanzpigmente. So sind ebenfalls bevorzugt Glasflakes deren mittlere geometrische Dicke in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich 200 nm bis 400 nm liegt.

In einer weiteren Ausführungsform liegt die mittlere geometrische Dicke für natürlichen oder synthetischen Glimmer als zu beschichtendes Substrat bevorzugt in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich von 200 nm bis 400 nm.

Beschichtet man plättchenförmige transparente Substrate unterhalb einer mittleren geometrischen Dicke von 50 nm mit hochbrechenden Metalloxiden, so werden extrem bruchempfindliche Mehrschichtperlglanzpigmente erhalten, die schon beim Einarbeiten in das Anwendungsmedium zerbrechen können, was wiederum eine signifikante Herabsetzung des Glanzes bedingt.

Oberhalb einer mittleren geometrischen Substratdicke von 5000 nm können die Mehrschichtperlglanzpigmente insgesamt zu dick werden. Damit geht eine schlechtere spezifische Deckfähigkeit, d.h. abgedeckte Fläche pro Gewichtseinheit an erfindungsgemäßem Mehrschichtperlglanzpigment, einher sowie eine geringere planparallele Orientierung im Anwendungsmedium. Aus einer schlechteren Orientierung wiederum resultiert ein verminderter Glanz.

Die mittlere geometrische Dicke des plättchenförmigen transparenten Substrates wird anhand eines gehärteten Lackfilmes, in dem die Mehrschichtperlglanzpigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die geometrische Dicke des plättchenförmigen transparenten Substrates von 100 Mehrschichtperlglanzpigmenten bestimmt und statistisch gemittelt wird.

Die erfindungsgemäßen Mehrschichtperlglanzpigmente werden hergestellt, indem die plättchenförmigen Substrate mit wenigstens einer optisch wirksamen Beschichtung versehen werden, welche

a) eine hochbrechende Schicht A mit einem Brechungsindex $n \geq 1,8$
b) eine niedrigbrechende Schicht B mit einem Brechungsindex $n < 1,8$
c) eine hochbrechende Schicht C mit einem Brechungsindex $n \geq 1,8$ sowie
d) optional eine äußere Schutzschicht D umfasst. Die Schichten A und B bzw. B und C können unter der äußeren Schutzschicht D mehrfach aufgebracht werden.

Bevorzugt werden immer alternierend hoch- und niedrigbrechende Schichten auf dem Substrat aufgebracht. Besonders bevorzugt wird das plättchenförmige transparente Substrat nur einmal mit den Schichten A bis C, optional D beschichtet.

Erfindungsgemäß ist die Schicht A in der Schichtenanordnung innenliegend, d.h. dem plättchenförmigen transparenten Substrat zugewandt, die Schicht B zwischen der Schicht A und der Schicht C liegend, und die Schicht C, bezogen auf das plättchenförmige transparente Substrat, in der Schichtenanordnung außenliegend.

Zwischen dem plättchenförmigen transparenten Substrat und der Schicht A können eine oder mehrere weitere, vorzugsweise im Wesentlichen transparente Schichten, angeordnet sein. Gemäß einer bevorzugten Weiterbildung ist die Schicht A direkt auf dem plättchenförmigen transparenten Substrat aufgebracht.

Zwischen der Schicht A und der Schicht B sowie zwischen der Schicht B und der Schicht C können unabhängig voneinander eine oder mehrere weitere, vorzugsweise im Wesentlichen transparente Schichten, angeordnet sein. Gemäß einer bevorzugten Weiterbildung ist die Schicht B direkt auf der Schicht A aufgebracht. Gemäß einer weiteren bevorzugten Weiterbildung ist die Schicht C direkt auf der Schicht B aufgebracht.

Äußerst bevorzugt ist die Schicht A unmittelbar auf dem plättchenförmigen transparenten Substrat, die Schicht B unmittelbar auf der Schicht A, die Schicht C unmittelbar auf der Schicht B sowie optional Schicht D unmittelbar auf der Schicht C aufgebracht.

Als optische wirksame Schichten oder Beschichtungen werden vorzugsweise Schichten aufgebracht, die Metalloxide, Metalloxidhydrate, Metallhydroxide, Metallsuboxide, Metalle, Metallfluoride, Metalloxyhalide, Metallchalcogenide, Metallnitride, Metalloxynitride, Metallsulfide, Metallcarbide oder Mischungen davon umfassen. Gemäß einer bevorzugten Variante bestehen die optisch wirksamen Schichten oder Beschichtungen aus den vorstehend genannten Materialien.

Die Begriffe Schichten oder Beschichtungen werden im Sinne dieser Erfindung austauschbar verwendet, sofern nicht anders angegeben.

Der Brechungsindex der hochbrechenden Schichten A und C liegt jeweils bei $n \geq 1,8$, bevorzugt bei $n \geq 1,9$ und besonders bevorzugt bei $n \geq 2,0$. Der Brechungsindex der niedrigbrechenden Schicht B liegt bei $n < 1,8$, bevorzugt bei $n < 1,7$ und besonders bevorzugt bei $n < 1,6$.

Für die hochbrechende Schicht A bzw. C eignen sich selektiv und/ oder nichtselektiv absorbierende und/ oder nicht absorbierende Materialien.

Als hochbrechende selektiv absorbierende Materialien eignen sich beispielsweise

- farbige Metalloxide oder Metalloxidhydrate wie Eisen(III)oxid ($\alpha$- und/ oder $\gamma$-$Fe_2O_3$, rot), FeO(OH) (gelb), Chrom (III)oxid (grün), Titan(III)oxid ($Ti_2O_3$, blau), Vanadiumpentoxid (orange),
- farbige Nitride wie Titanoxynitride und Titannitrid ($TiO_xN_y$, TiN, blau), wobei die niederen Titanoxide und -nitride in der Regel im Gemisch mit Titandioxid vorliegen,
- Metallsulfide wie Cersulfid (rot),
- Eisentitanate wie Pseudobrookit (braunrot) und/ oder Pseudorutil (braunrot),
- Zinn-Antimonoxid $Sn(Sb)O_2$,
- nicht absorbierende farblose hochbrechende Materialien, z.B. Metalloxide wie Titandioxid und Zirkoniumdioxid, die mit selektiv absorbierenden Farbmitteln eingefärbt sind. Diese Einfärbung kann durch Einbau von Farbmitteln in die Metalloxidschicht, durch deren Dotierung mit selektiv absorbierenden Metallkationen oder farbigen Metalloxiden wie Eisen(III)oxid oder durch Überziehen der Metalloxidschicht mit einem ein Farbmittel enthaltenden Film erfolgen.

Beispiele für hochbrechende nichtselektiv absorbierende Materialien sind u.a.

- Metalle wie Molybdän, Eisen, Wolfram, Chrom, Cobalt, Nickel, Silber, Palladium, Platin, deren Gemische oder Legierungen,
- Metalloxide wie Magnetit $Fe_3O_4$, Cobaltoxid (CoO und/ oder $Co_30_4$), Vanadiumoxid ($VO_2$ und/ oder $V_2O_3$) sowie auch Mischungen dieser Oxide mit Metallen, insbesondere Magnetit und (metallischem) Eisen,
- Eisentitanante wie Ilmenit,
- Metallsulfide wie Moybdänsulfid, Eisensulfid, Wolframsulfid, Chromsulfid, Colbaltsulfid, Nickelsulfid, Silbersulfid, Zinnsulfid, Gemische dieser Sulfide, Gemische dieser Sulfide mit dem jeweiligen Metall, wie $MoS_2$ und Mo, und Gemische mit Oxiden des jeweiligen Metalls, wie $MoS_2$ und Molybdänoxide,
- nicht absorbierende farblose hochbrechende Schichten wie Titandioxid oder Zirkoniumdioxid, in die nichtselektiv absorbierendes Material (z.B. Kohlenstoff) eingebaut ist oder die damit beschichtet sind.

Zu den hochbrechenden nicht absorbierenden Materialien zählen beispielsweise

- Metalloxide wie Titandioxid, Zirkoniumdioxid, Zinkoxid, Zinndioxid, Antimonoxid und deren Gemische,
- Metallhydroxide,
- Metalloxidhydrate,
- Metallsulfide wie Zinksulfid,
- Metalloxyhalide wie Bismutoxychlorid.

Die Schichten A und/ oder C können jeweils auch Mischungen verschiedener selektiv und/ oder nicht selektiv absorbierender Komponenten, vorzugsweise Metalloxide, sein. Beispielsweise können die verschiedenen Komponenten, vorzugsweise Metalloxide, als homogene Mischung vorliegen. Es ist aber auch möglich, dass die eine Komponente in der anderen Komponente in Form einer Dotierung vorliegt.

Beispielsweise kann in der Schicht A und/ oder C eine nicht absorbierende Komponente, beispielsweise Titanoxid, vorzugsweise $TiO_2$, als Dotierung in einer selektiv absorbierenden Komponente, vorzugsweise $Fe_2O_3$, und/

oder einer nicht selektiv absorbierenden Komponente, beispielsweise $Fe_3O_4$, vorliegen. Alternativ kann auch eine selektiv absorbierende Komponente, beispielsweise $Fe_2O_3$, und/ oder eine nicht selektiv absorbierende Komponente, beispielsweise $Fe_3O_4$, als Dotierung in einer nicht absorbierenden Komponente, beispielsweise Titanoxid, vorzugsweise $TiO_2$, vorliegen.

In einer bevorzugten Ausführungsform werden als hochbrechende Schicht A bzw. C Metalloxide, Metallhydroxide und/ oder Metalloxidhydrate verwendet. Besonders bevorzugt werden Metalloxide eingesetzt. Ganz besonders bevorzugt umfasst die Schicht A und/ oder C Titandioxid und/ oder Eisenoxid sowie Mischungen davon. Bei einer Ausführungsform besteht die Schicht A und/ oder C aus Titandioxid und/ oder Eisenoxid sowie Mischungen davon.

Weisen die erfindungsgemäßen Mehrschichtperlglanzpigmente eine Beschichtung mit Titandioxid auf, kann das Titandioxid in der Rutil- oder Anataskristallmodifikation vorliegen. Vorzugsweise liegt die Titandioxidschicht in der Rutilform vor. Die Rutilform kann erhalten werden, indem beispielsweise vor Aufbringung der Titandioxidschicht eine Schicht aus Zinndioxid auf das zu beschichtende plättchenförmige transparente Substrat aufgebracht wird. Auf dieser Schicht aus Zinndioxid kristallisiert Titandioxid in der Rutilmodifikation. Das Zinndioxid kann dabei als separate Schicht vorliegen, wobei die Schichtdicke wenige Nanometer, beispielsweise weniger als 10 nm, weiter bevorzugt weniger als 5 nm, noch weiter bevorzugt weniger als 3 nm, betragen kann.

Als niedrigbrechende Schicht B eigenen sich nicht absorbierende Materialien. Hierzu zählen beispielsweise

- Metalloxide wie Siliziumdioxid, Aluminiumoxid, Boroxid,
- Metalloxidhydrate wie Siliziumoxidhydrat, Aluminiumoxidhydrat,
- Metallfluoride wie Magnesiumfluorid,
- $MgSiO_3$.

Gegebenenfalls kann die niedrigbrechende Metalloxidschicht Alkali- und/ oder Erdalkalioxide als Bestandteile enthalten.

Vorzugsweise umfasst die niedrigbrechende Schicht B Siliziumdioxid. Bei einer Ausführungsform besteht die niedrigbrechende Schicht B aus Siliziumdioxid.

Die interferenzfähige Beschichtung kann entweder das Substrat vollständig umhüllen oder nur partiell auf dem Substrat vorliegen. Die erfindungsgemäßen Mehrschichtperlglanzpigmente zeichnen sich durch den gleichmäßigen homogenen Aufbau der Beschichtung aus, welche das plättchenförmige Substrat vollständig umhüllt und nicht nur dessen Ober- und Unterseite bedeckt.

Die optische Dicke der nichtmetallischen Schichten mit hoher und niedriger Brechzahl bestimmt die optischen Eigenschaften der Mehrschichtperlglanzpigmente. Bei den erfindungsgemäßen Mehrschichtperlglanzpigmenten mit silberner Interferenzfarbe wird vorzugsweise die Dicke der einzelnen Schichten aufeinander abgestimmt.

Wenn n die Brechzahl einer Schicht und d ihre Dicke ist, ergibt sich die Interferenzfarbe, in der eine dünne Schicht erscheint, aus dem Produkt von n und d, d.h. der optischen Dicke. Die bei Normallichteinfall im reflektierenden Licht entstehenden Farben eines solchen Filmes ergeben sich aus einer Verstärkung des Lichtes der Wellenlänge

$$\lambda = \frac{4}{2N-1} \cdot nd$$

und durch Schwächung von Licht der Wellenlänge

$$\lambda = \frac{2}{N} \cdot nd ,$$

wobei N eine positive ganze Zahl ist. Die bei zunehmender Filmdicke erfolgende Variation der Farbe ergibt sich aus der Verstärkung bzw. Schwächung bestimmter Wellenlängen des Lichtes durch Interferenz.

Bei mehrschichtigen Pigmenten wird die Interferenzfarbe durch die Verstärkung bestimmter Wellenlängen bestimmt und wenn mehrere Schichten in einem vielschichtigen Pigment die gleiche optische Dicke besitzen, wird die Farbe des reflektierenden Lichtes mit zunehmender Zahl der Schichten intensiver.

Die erfindungsgemäßen Mehrschichtpigmente mit silberner Interferenzfarbe können, optische Schichtdicken der hochbrechenden Schichten A und C aufweisen, die jeweils im Bereich von 50 nm bis 200 nm, bevorzugt im Bereich von 95 nm bis 180 nm und besonders bevorzugt im Bereich von 110 nm bis 165 nm liegen. Die optische Schichtdicke der niedrigbrechenden Schicht B kann in einem Bereich von 30 nm bis 150 nm, bevorzugt in einem Bereich von 70 nm bis 150 nm und besonders bevorzugt in einem Bereich von 100 nm bis 150 nm liegen.

Im Fall von Metallen liegen die geometrischen Schichtdicken der Schicht A bzw. C bei 10 nm bis 50 nm, bevorzugt bei 15 nm bis 30 nm. Die Schichtdicken müssen so eingestellt werden, dass die Schichten semitransparente Eigenschaften aufweisen. Abhängig vom verwendeten Metall kann das zu unterschiedlichen Schichtdicken führen.

Die Beschichtung soll für sichtbares Licht zumindest teilweise durchlässig (semitransparent) sein und ist daher in Abhängigkeit von den optischen Eigenschaften der ausgewählten Schichtmaterialien unterschiedlich dick.

Bei den in dieser Anmeldung angegebenen Schichtdicken handelt es sich - sofern nicht anders angegeben - um die optischen Schichtdicken. Unter einer optischen Schichtdicke wird erfindungsgemäß das Produkt aus geometrischer Schichtdicke und dem Brechungsindex des die Schicht aufbauenden Materials verstanden. Als Wert für den Brechungsindex des jeweiligen Materials wird der aus der Literatur jeweils bekannte Wert verwendet. Die geometrische Schichtdicke wird dabei erfindungsgemäß anhand von Querschliffaufnahmen von Lacken, in denen planparallel zum Untergrund orientierte Mehrschichtperlglanzpigmente vorliegen, mittels REM bestimmt.

Die optischen Schichtdicken der erfindungsgemäßen Mehrschichtperlglanzpigmente können derart eingestellt sein, dass sowohl Schicht A als auch Schicht C für sich genommen bereits einen silbernen Eindruck hervorruft. Alternativ können diese beiden Schichten jeweils für sich betrachtet auch einen nichtsilbernen Eindruck aufweisen. Wichtig ist, dass die Interferenzfarbe der komplett beschichteten erfindungsgemäßen Mehrschichtperlglanzpigmente silber ist.

Unter Mehrschichtperlglanzpigmenten mit silberner Interferenzfarbe werden im Rahmen dieser Erfindung Mehrschichtperlglanzpigmente verstanden, deren Chromawerte $C^*_{15} \leq 20$, vorzugsweise $\leq 19$, weiter bevorzugt $\leq 18$ und besonders bevorzugt $\leq 17$ sind. Die Chromawerte $C^*_{15}$ der erfindungsgemäßen Mehrschichtperlglanzpigmente liegen bevorzugt in einem Bereich von 1 bis $\leq 20$, weiter bevorzugt in einem Bereich von 2 bis $\leq 19$, weiter bevorzugt in einem Bereich von 3 bis $\leq 18$ und besonders bevorzugt in einem Bereich von 4 bis $\leq 17$. Die Mehrschichtperlglanzpigmente können hierbei neben ihrer silbernen Interferenzfarbe bei einer Betrachtung außerhalb des Glanzwinkels einen farbigen Eindruck aufweisen. Dieser kann je nach Art der Beschichtung des plättchenförmigen transparenten Substrats durch die Eigenfarbe des Beschichtungsmittels und durch dessen Schichtdicke hervorgerufen werden. Aufgrund des hohen Glanzes wird im Glanzwinkel eine möglicherweise vorhandene Absorptionsfarbe überstrahlt und der Gesamteindruck des erfindungsgemäßen Mehrschichtperlglanzpigmentes vermittelt sich dem Betrachter als silberfarben. In weiteren Ausführungsformen kann die silberne Interferenzfarbe im Glanzwinkel auch einen leicht pastellfarbigen Farbton aufweisen, jedoch nimmt der Beobachter hier einen überwiegend silbernen Farbeindruck war.

Die Chromawerte werden dabei anhand von folgenden Applikationen ermittelt: Ein Nitrocelluloselack (Dr. Renger Erco Bronzemischlack 2615e; Fa. Morton), der 6 Gew.-% an Mehrschichtperlglanzpigmenten enthält, wobei sich die Gew.-% Angabe auf das Gesamtgewicht des Lackes bezieht, wird je nach $D_{50}$-Wert in einer Nassfilmdicke gemäß Tabelle 3 auf BYK-Gardner Schwarz-Weiß-Rakelkarten (Byko-Chart 2853) aufgetragen und nachfolgend bei Raumtemperatur getrocknet. An diesen Rakelkarten werden dann mit einem BYK-MAC (Fa. BYK Gardner) farbmetrische Auswertungen vorgenommen, wobei auf dem schwarzen Untergrund der Rakelkarte vermessen wird. Der Einfallswinkel beträgt 45° und herangezogen wird der Chromawert bei einem Beobachtungswinkel von 15°.

Tabelle 3: Nassfilmdicke in Abhängigkeit vom $D_{50}$-Wert der Mehrschichtperlglanzpigmente

| $D_{50}$-Wert | Spiralrakel |
|---|---|
| <40 µm | 36 µm |
| 40 µm - 85 µm | 76 µm |
| > 85 µm | 100 µm |

Die erfindungsgemäßen Mehrschichtperlglanzpigmente sind nicht goniochromatisch, d.h. sie weisen keinen winkelabhängigen Farbwechsel der Interferenzfarbe auf.

Die Mehrschichtperlglanzpigmente können weiterhin mit wenigstens einer äußeren Schutzschicht D versehen sein, die die Licht-, Wetter-, und/ oder chemische Stabilität des Mehrschichtperlglanzpigmentes weiter erhöht. Bei der äußeren Schutzschicht D kann es sich auch um eine Nachbeschichtung handeln, die die Handhabung des Pigments bei Einarbeitung in unterschiedliche Medien erleichtert.

Die äußere Schutzschicht D der erfindungsgemäßen Mehrschichtperlglanzpigmente kann eine oder zwei Metalloxidschichten der Elemente Si, Al oder Ce umfassen bzw. bevorzugt daraus bestehen. Bei einer Variante wird als äußerste Metalloxidschicht eine Siliziumoxidschicht, vorzugsweise $SiO_2$-Schicht, aufgebracht. Besonders bevorzugt ist hierbei eine Reihenfolge, bei der zunächst eine Ceroxidschicht aufgebracht ist, der dann eine $SiO_2$-Schicht folgt, wie in der WO 2006/021386 A1 beschrieben, deren Inhalt hiermit unter Bezugnahme aufgenommen ist.

Die äußere Schutzschicht D kann des Weiteren an der Oberfläche organischchemisch modifiziert sein. Beispielsweise können ein oder mehrere Silane auf dieser äußeren Schutzschicht aufgebracht sein. Bei den Silanen kann es sich um Alkylsilane mit verzweigten oder unverzweigten Alkylresten mit 1 bis 24 C-Atomen, vorzugsweise 6 bis 18 C-Atomen handeln.

Bei den Silanen kann es sich aber auch um organofunktionelle Silane handeln, die eine chemische Anbindung an einen Kunststoff, ein Bindemittel eines Lackes oder einer Farbe, etc. ermöglichen.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Evonik hergestellt und unter dem Handelsnamen "Dynasylan" vertrieben. Weitere Produkte können von der Fa. Momentive (Silquest-Silane) oder von der Fa. Wacker, beispielsweise Standard- und $\alpha$-Silane aus der GENIOSIL-Produktgruppe, bezogen werden. Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), Methyltri(m)ethoxysilan (Dynasylan MTMS bzw. MTES), 3-Mercaptopropyltrimethoxysilan (Dynasylan MTMO; Silquest A-189), 3-Glycidoxypropyltrimethoxysilan (Dynasylan GLYMO, Silquest A-187), Tris[3-(trimethoxysilyl)propyl]isocyanurat (Silquest Y-11597), Bis[3-(triethoxysilyl)propyl)]tetrasulfid (Silquest A-1289), Bis[3-(triethoxysilyl)propyldisulfid (Silquest A-1589), beta-(3,4-Epoxycyclohexyl) ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), (Isocyanatomethyl)methyldimethoxysilan, (Isocyanatomethyl)trimethoxysilan, 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltriacetoxysilan, 3- Methacryloxypropylmethyldimethoxysilan, Vinyltrichlorsilan, Vinyltrimethoxysilan (GENIOSIL XL 10), Vinyltris(2-methoxyethoxy) silan (GENIOSIL GF 58), Vinyltriacetoxysilan.

Vorzugsweise werden als organofunktionelle Silane 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), Methyltri(m) ethoxysilan (Dynasylan MTMS bzw. MTES), beta-(3,4-Epoxycyclohexyl)ethyltrimethoxysilan (Silquest A-186), Bis (triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), 3-(Triethoxysilyl)propylbernsteinsäureanhydrid (GENIOSIL GF 20), Vinyltrimethoxysilan (GENIOSIL XL 10) und/ oder Vinyltris(2-methoxyethoxy)silan (GENIOSIL GF 58) verwendet.

Es ist aber auch möglich, andere organofunktionelle Silane auf die erfindungsgemäßen Mehrschichtperlglanzpigmente aufzubringen.

Weiterhin lassen sich, beispielsweise kommerziell von Degussa erhältliche, wässrige Vorhydrolysate einsetzen. Hierzu gehören u.a. wässriges Aminosiloxan (Dynasylan Hydrosil 1151), wässriges amino-/ alkylfunktionelles Siloxan (Dynasylan Hydrosil 2627 oder 2909), wässriges diaminofunktionelles Siloxan (Dynasylan Hydrosil 2776), wässriges, wässriges epoxyfunktionelles Siloxan (Dynasylan Hydrosil 2926), amino-/ alkylfunktionelles Oligosiloxan (Dynasylan 1146), vinyl-/ alkylfunktionelles Oligosiloxan (Dynasylan 6598), oligomeres Vinylsilan (Dynasylan 6490) oder oligomeres kurzkettiges alkylfunktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Mehrschichtperlglanzpigments an verschiedenartige Bindemittel sehr gut geeignet.

Bevorzugt werden hierzu folgende Verbindungen genommen: 3-Aminopropyltrimethoxysilan (Dynasylan AMMO; Silquest A-1110), 3-Aminopropyltriethoxysilan (Dynasylan AMEO), [3-(2-Aminoethyl)-aminopropyl]trimethoxysilan (Dynasylan DAMO, Silquest A-1120), [3-(2-Aminoethyl)-aminopropyl]triethoxysilan, triaminofunktionelles Trimethoxysilan (Silquest A-1130), Bis-(gamma-trimethoxysilylpropyl)amin (Silquest A-1170), N-Ethyl-gammaaminoisobutyltrimethoxysilan (Silquest A-Link 15), N-Phenyl-gamma-aminopropyltrimethoxysilan (Silquest Y-9669), 4-Amino-3,3-dimethylbutyltrimethoxysilan (Silquest A-1637), N-Cyclohexylaminomethylmethyldiethoxysilan (GENIOSIL XL 924), N-Cyclohexylaminomethyltriethoxysilan (GENIOSIL XL 926), N-Phenylaminomethyltrimethoxysilan (GENIOSIL XL 973) und deren Mischungen.

Bei einer weiterhin bevorzugten Ausführungsform ist das Silan ohne funktionelle Bindungsgruppe ein Alkylsilan. Das Alkylsilan weist vorzugsweise die Formel (A) auf:

$$R_{(4-z)}Si(X)_z, \qquad (A)$$

Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/ oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

An oder auf der Oberfläche der erfindungsgemäßen Mehrschichtperlglanzpigmente können neben den erwähnten Silanen bzw. Silangemischen auch weitere organischchemische Modifizierungsmittel, wie beispielsweise substituierte oder nicht substituierte Alkylreste, Polyether, Thioether, Siloxane, etc. und Mischungen davon angeordnet sein. Es können aber auch anorganisch-chemische Modifizierungsmittel (z.B. $Al_2O_3$ oder $ZrO_2$ oder deren Gemische), welche z.B. die Dispergierbarkeit und/ oder Verträglichkeit im jeweiligen Anwendungsmedium erhöhen können, auf die Pigmentoberfläche aufgebracht werden.

Über die Oberflächenmodifizierung kann beispielsweise die Hydrophilie oder Hydrophobie der Pigmentoberfläche verändert und/ oder eingestellt werden. Beispielsweise können über die Oberflächenmodifizierung die Leafing-bzw. Nonleafing-Eigenschaften der erfindungsgemäßen Mehrschichtperlglanzpigmente verändert und/ oder eingestellt werden. Unter Leafing wird verstanden, dass sich die erfindungsgemäßen Mehrschichtperlglanzpigmente in einem Anwendungsmedium, beispielsweise einem Lack oder einer Druckfarbe, an oder in der Nähe der Grenz- oder Oberfläche des Anwendungsmediums anordnen.

Die Oberflächenmodifizierungsmittel können auch reaktive chemische Gruppen, wie beispielsweise Acrylat-, Methacrylat-, Vinyl-, Isocyanat-, Cyano-, Epoxy-, Hydroxy-, Aminogruppen oder Mischungen davon, aufweisen. Diese chemisch reaktiven Gruppen ermöglichen eine chemische Anbindung, insbesondere Ausbildung von kovalenten Bindungen, an das Anwendungsmedium oder Komponenten des Anwendungsmediums, wie beispielsweise Bindemittel. Hierdurch können beispielsweise die chemischen und/ oder physikalischen Eigenschaften von ausgehärteten Lacken, Farben oder Druckfarben wie Beständigkeit gegenüber Umwelteinflüssen wie Feuchtigkeit, Sonneneinstrahlung, UV-Beständigkeit, etc., oder gegenüber mechanischen Einflüssen, beispielsweise Kratzern etc., verbessert werden.

Die chemische Reaktion zwischen den chemisch-reaktiven Gruppen und dem Anwendungsmedium bzw. Komponenten des Anwendungsmediums kann beispielsweise durch Einstrahlung von Energie, beispielsweise in Form von UV-Strahlung und/ oder Wärme, induziert werden.

Für die Einarbeitung von mit Silanen nachbeschichteten bzw. mit einer äußeren Schutzschicht D versehenen Mehrschichtperlglanzpigmenten in kosmetischen Formulierungen muss darauf geachtet werden, dass das entsprechende Silan bzw. das Material der äußeren Schutzschicht D nach der Kosmetikverordnung zulässig ist.

Bei einer weiteren Ausführungsform umfasst die vorliegende Erfindung Mehrschichtperlglanzpigmente mit silberner Interferenzfarbe basierend auf plättchenförmigen transparenten Substraten mit optisch wirksamer Beschichtung, wobei die optisch wirksame Beschichtung wenigstens

a) eine hochbrechende, nicht absorbierende Schicht A mit einem Brechungsindex $n \geq 1,8$
b) eine niedrigbrechende, nicht absorbierende Schicht B mit einem Brechungsindex $n < 1,8$
c) eine hochbrechende, nicht absorbierende Schicht C mit einem Brechungsindex $n \geq 1,8$
sowie
d) optional eine äußere Schutzschicht D

umfasst und die Mehrschichtperlglanzpigmente die Kennzahlen $D_{10}$, $D_{50}$, $D_{90}$ aus der Summenhäufigkeitsverteilung der volumengemittelten Größenverteilungsfunktion mit einem Span $\Delta D$ von 0,8 -1,2 aufweisen, wobei der Span $\Delta D$ gemäß der Formel $\Delta D = (D_{90}- D_{10})/ D_{50}$ berechnet ist.

Bei einer weiteren Ausführungsform umfasst die vorliegende Erfindung Mehrschichtperlglanzpigmente mit silberner Interferenzfarbe basierend auf plättchenförmigen transparenten Substraten mit optisch wirksamer Beschichtung, wobei die optisch wirksame Beschichtung wenigstens

e) eine hochbrechende, nicht absorbierende Schicht A mit einem Brechungsindex $n \geq 1,8$
f) eine niedrigbrechende, nicht absorbierende Schicht B mit einem Brechungsindex $n < 1,8$
g) eine hochbrechende, selektiv absorbierende Schicht C mit einem Brechungsindex $n \geq 1,8$
sowie
h) optional eine äußere Schutzschicht D

umfasst und die Mehrschichtperlglanzpigmente die Kennzahlen $D_{10}$, $D_{50}$, $D_{90}$ aus der Summenhäufigkeitsverteilung der volumengemittelten Größenverteilungsfunktion mit einem Span $\Delta D$ von 0,8 - 1,2 aufweisen, wobei der Span $\Delta D$ gemäß der Formel $\Delta D = (D_{90} - D_{10})/ D_{50}$ berechnet ist.

Bei einer weiteren Ausführungsform umfasst die vorliegende Erfindung Mehrschichtperlglanzpigmente mit silberner Interferenzfarbe basierend auf plättchenförmigen transparenten Substraten mit optisch wirksamer Beschichtung, wobei die optisch wirksame Beschichtung wenigstens

i) eine hochbrechende, selektiv absorbierende Schicht A mit einem Brechungsindex $n \geq 1,8$
j) eine niedrigbrechende, nicht absorbierende Schicht B mit einem Brechungsindex $n < 1,8$

k) eine hochbrechende, nicht absorbierende Schicht C mit einem Brechungsindex n ≥ 1,8
sowie
l) optional eine äußere Schutzschicht D

umfasst und die Mehrschichtperlglanzpigmente die Kennzahlen $D_{10}$, $D_{50}$, $D_{90}$ aus der Summenhäufigkeitsverteilung der volumengemittelten Größenverteilungsfunktion mit einem Span ΔD von 0,8 - 1,2 aufweisen, wobei der Span ΔD gemäß der Formel $\Delta D = (D_{90} - D_{10}) / D_{50}$ berechnet ist.

Bei einer weiteren Ausführungsform umfasst die vorliegende Erfindung Mehrschichtperlglanzpigmente mit silberner Interferenzfarbe basierend auf plättchenförmigen transparenten Substraten mit optisch wirksamer Beschichtung, wobei die optisch wirksame Beschichtung wenigstens

m) eine hochbrechende, selektiv absorbierende Schicht A mit einem Brechungsindex n ≥ 1,8
n) eine niedrigbrechende, nicht absorbierende Schicht B mit einem Brechungsindex n < 1,8
o) eine hochbrechende, selektiv absorbierende Schicht C mit einem Brechungsindex n ≥ 1,8
sowie
p) optional eine äußere Schutzschicht D

umfasst und die Mehrschichtperlglanzpigmente die Kennzahlen $D_{10}$, $D_{50}$, $D_{90}$ aus der Summenhäufigkeitsverteilung der volumengemittelten Größenverteilungsfunktion mit einem Span ΔD von 0,8 - 1,2 aufweisen, wobei der Span ΔD gemäß der Formel $\Delta D = (D_{90} - D_{10}) / D_{50}$ berechnet ist.

Bei einer weiteren Ausführungsform umfasst die vorliegende Erfindung Mehrschichtperlglanzpigmente mit silberner Interferenzfarbe basierend auf plättchenförmigen transparenten Substraten mit optisch wirksamer Beschichtung, wobei die optisch wirksame Beschichtung wenigstens

q) eine hochbrechende, nicht absorbierende Schicht A mit einem Brechungsindex n ≥ 1,8
r) eine niedrigbrechende, nicht absorbierende Schicht B mit einem Brechungsindex n < 1,8
s) eine hochbrechende, nichtselektiv absorbierende Schicht C mit einem Brechungsindex n ≥ 1,8, umfassend oder bestehend aus beispielsweise Magnetit oder Ilmenit
sowie
t) optional eine äußere Schutzschicht D

umfasst und die Mehrschichtperlglanzpigmente die Kennzahlen $D_{10}$, $D_{50}$, $D_{90}$ aus der Summenhäufigkeitsverteilung der volumengemittelten Größenverteilungsfunktion mit einem Span ΔD von 0,8 -1,2 aufweisen, wobei der Span ΔD gemäß der Formel $\Delta D = (D_{90} - D_{10}) / D_{50}$ berechnet ist.

Bei einer weiteren Ausführungsform umfasst die optisch wirksame Beschichtung A bis C der erfindungsgemäßen Mehrschichtperlglanzpigmente folgende Kombinationen:

Tabelle 4: Ausführungsform der optisch wirksamen Beschichtung

| Schicht A | Schicht B | Schicht C |
|---|---|---|
| $TiO_2$ | $SiO_2$ | $TiO_2$ |
| $TiO_2$ | $SiO_2$ | $Fe_2O_3$ |
| $Fe_2O_3$ | $SiO_2$ | $TiO_2$ |
| $Fe_2O_3$ | $SiO_2$ | $Fe_2O_3$ |

Bei einer weiteren Ausführungsform besteht die optisch wirksame Beschichtung der erfindungsgemäßen Mehrschichtperlglanzpigmente aus in Tabelle 4 genannten Kombinationen.

In einer weiteren Ausführungsform umfasst die vorliegende Erfindung Mehrschichtperlglanzpigmente mit silberner Interferenzfarbe basierend auf plättchenförmigen transparenten Substraten mit optisch wirksamer Beschichtung, wobei die optisch wirksame Beschichtung wenigstens

u) eine hochbrechende Schicht A mit einem Brechungsindex n ≥ 1,8 und einer optischen Schichtdicke von 50 bis 200 nm
v) eine niedrigbrechende Schicht B mit einem Brechungsindex n < 1,8 und einer optischen Schichtdicke von 30 bis 150 nm
w) eine hochbrechende Schicht C mit einem Brechungsindex n ≥ 1,8 und einer optischen Schichtdicke von 50 bis 200 nm
sowie
x) optional eine äußere Schutzschicht D

umfasst und die Mehrschichtperlglanzpigmente die Kennzahlen $D_{10}$, $D_{50}$, $D_{90}$ aus der Summenhäufigkeitsverteilung der volumengemittelten Größenverteilungsfunktion mit einem Span $\Delta D$ von 0,8 -1,2 aufweisen, wobei der Span $\Delta D$ gemäß der Formel $\Delta D = (D_{90} - D_{10})/ D_{50}$ berechnet ist.

Bei einer weiteren Ausführungsform umfasst die vorliegende Erfindung Mehrschichtperlglanzpigmente mit silberner Interferenzfarbe basierend auf plättchenförmigen transparenten Substraten mit optisch wirksamer Beschichtung, wobei die optisch wirksame Beschichtung wenigstens

y) eine hochbrechende Schicht A mit einem Brechungsindex $n \geq 1,8$ und einer optischen Schichtdicke von 50 bis 200 nm
z) eine niedrigbrechende Schicht B mit einem Brechungsindex $n < 1,8$ und einer optischen Schichtdicke von 30 bis 150 nm
aa) eine hochbrechende Schicht C mit einem Brechungsindex $n \geq 1,8$ und einer optischen Schichtdicke von 50 bis 200 nm
sowie
bb) optional eine äußere Schutzschicht D

umfasst und die Mehrschichtperlglanzpigmente die Kennzahlen $D_{10}$, $D_{50}$, $D_{90}$ aus der Summenhäufigkeitsverteilung der volumengemittelten Größenverteilungsfunktion mit einem Span $\Delta D$ von 0,8 -1,2 aufweisen, wobei der Span $\Delta D$ gemäß der Formel $\Delta D = (D_{90} - D_{10})/ D_{50}$ berechnet ist und das Chroma $C^*_{15}$ der Mehrschichtperlglanzpigmente $\leq 20$, bevorzugt $\leq 17$ ist.

Ein Verfahren zur Herstellung der Mehrschichtperlglanzpigmente mit silberner Interferenzfarbe umfasst die folgenden Schritte:

i) Größenklassieren der zu beschichtenden plättchenförmigen transparenten Substrate
ii) Beschichten der plättchenförmigen transparenten Substrate mit oben genannten Schichten A bis C sowie optional Schicht D.

Wenn die Ausgangssubstrate zu groß sind, kann optional ein Zerkleinerungsschritt vor dem Größenklassieren durchgeführt werden.

Die Größenklassierung kann vor oder nach der Beschichtung der Substrate erfolgen. Vorteilhafterweise wird jedoch zuerst das Substrat klassiert und anschließend beschichtet. Die Größenklassierung wird durchgeführt und gegebenenfalls wiederholt, bis die Mehrschichtperlglanzpigmente die erfindungsgemäße Größenverteilung aufweisen.

Ein enger Span $\Delta D$ der Substrate kann durch geeignete Zerkleinerungs- und/ oder Klassierungsprozesse der zu beschichtenden plättchenförmigen transparenten Substrate erreicht werden. Die zu beschichtenden plättchenförmigen transparenten Substrate können beispielsweise durch Kugelmühle, Strahl- oder Rührwerkskugelmühle, Kollergang oder Dissolver zerkleinert werden. Der Span $\Delta D$ der Endfraktion kann durch geeignete Klassierung, wie beispielsweise eine mehrfache Nasssiebung, eingestellt werden. Weitere Klassierungsverfahren umfassen die Zentrifugation in Zyklonen oder eine Sedimentation aus einer Dispersion.

Die Zerkleinerungs- und Klassierprozesse können nacheinander erfolgen und gegebenenfalls miteinander kombiniert werden. So kann auf einen Zerkleinerungsvorgang ein Klassierungsvorgang folgen, dem sich ein weiterer Zerkleinerungsvorgang des Feinguts anschließt usw.

Die Metalloxidschichten werden vorzugsweise nasschemisch aufgebracht, wobei die zur Herstellung von Perlglanzpigmenten entwickelten nasschemischen Beschichtungsverfahren angewendet werden können. Bei der Nassbeschichtung werden die Substratpartikel in Wasser suspendiert und mit einem oder mehreren hydrolysierbaren Metallsalzen oder einer Wasserglaslösung bei einem für die Hydrolyse geeigneten pH-Wert versetzt, der so gewählt wird, dass die Metalloxide bzw. Metalloxidhydrate direkt auf dem zu beschichtenden Substrat aufgefällt werden, ohne dass es zu Nebenfällungen kommt. Der pH-Wert wird üblicherweise durch gleichzeitiges Zudosieren einer Base und/ oder einer Säure konstant gehalten. Anschließend werden die Pigmente abgetrennt, gewaschen und bei 50 - 150°C für 6 - 18 Stunden getrocknet und gegebenenfalls 0,5 - 3 Stunden geglüht, wobei die Glühtemperatur im Hinblick auf die jeweils vorliegende Beschichtung optimiert werden kann. In der Regel liegen die Glühtemperaturen zwischen 500 und 1000°C, vorzugsweise zwischen 600 und 900°C. Falls gewünscht können die Pigmente nach Aufbringen einzelner Beschichtungen abgetrennt, getrocknet und gegebenenfalls geglüht werden, um dann zur Auffällung der weiteren Schichten wieder resuspendiert zu werden.

Die Auffällung der $SiO_2$-Schicht auf das zu beschichtende plättchenförmige transparente Substrat kann durch Zugabe einer Kalium- oder Natronwasserglaslösung bei einem geeigneten pH-Wert erfolgen. Alternativ kann die $SiO_2$-Schicht über Sol-Gel-Verfahren ausgehend von Alkoxysilanen, wie z.B. Tetraethoxysilan, aufgebracht werden.

Die erfindungsgemäßen Mehrschichtperlglanzpigmente lassen sich auch vorteilhaft in Abmischungen mit transparenten und deckenden Weiß-, Bunt- und Schwarzpigmenten sowie weiteren Effektpigmenten verwenden.

Die erfindungsgemäßen Mehrschichtperlglanzpigmente können zur Herstellung von Pigmentpräparationen und Trockenpräparaten eingesetzt werden.

Weiterhin können die erfindungsgemäßen Mehrschichtperlglanzpigmente beispielsweise in kosmetischen Formulierungen, Kunststoffen, keramischen Materialien, Gläsern und Beschichtungszusammensetzungen wie Farben, Druckfarben, z.B. für den Offset-, Sieb-, Tief-, Flexo-, Sicherheitsdruck oder Bronzierung, Tinten, in Tonern, Lacken, z.B. Autolacken oder Pulverlacken, zur Lasermarkierung von Papier und Kunststoffen, zur Saatguteinfärbung, zur Einfärbung von Lebensmitteln oder pharmazeutischen Erzeugnissen oder zur Farbgebung von (Agrar-)Folien, Zeltplanen oder Textilien verwendet werden.

In kosmetischen Formulierungen können die erfindungsgemäßen Mehrschichtperlglanzpigmente mit silberner Interferenzfarbe mit für die jeweilige Anwendung geeigneten Roh-, Hilfs- und Wirkstoffen kombiniert werden. Die Konzentration der Mehrschichtperlglanzpigmente in der Formulierung kann zwischen 0,001 Gew.-% für Rinse-off-Produkte und 40,0 Gew.-% für Leave-on-Produkte liegen.

Die erfindungsgemäßen Mehrschichtperlglanzpigmente eignen sich insbesondere für die Anwendung in Kosmetika, wie z.B. Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semipermanente Haarfarben, temporäre Haarfarben, Hautpflegekompositionen wie Lotions, Gele, Emulsionen sowie Nagellackkompositionen.

Zur Erzielung spezieller Farbeffekte können in den Kosmetikapplikationen neben den erfindungsgemäßen Mehrschichtperlglanzpigmenten weitere Farbmittel und/ oder herkömmliche Effektpigmente oder Mischungen hiervon in variablen Mengenverhältnissen eingesetzt werden. Als herkömmliche Effektpigmente können beispielsweise handelsübliche Perlglanzpigmente auf der Basis von mit hochbrechenden Metalloxiden beschichtetem natürlichen Glimmer (wie z.B. die Produktgruppe Prestige der Fa. Eckart), BiOCl-Plättchen, $TiO_2$-Plättchen, Perlglanzpigmente auf der Basis von mit hochbrechenden Metalloxiden beschichtetem synthetischen Glimmer oder basierend auf mit hochbrechenden Metalloxiden beschichteten Glasplättchen (wie z.B. die Produktgruppe MIRAGE der Fa. Eckart), $Al_2O_3$-, $SiO_2$- oder $TiO_2$-Plättchen verwendet werden. Außerdem können auch Metalleffektpigmente, wie z.B. die Produktgruppe Visionaire der Fa. Eckart, zugegeben werden. Die Farbmittel können aus anorganischen oder organischen Pigmenten ausgewählt sein.

Im Folgenden wird die Erfindung durch einige Beispiele näher erläutert, ohne auf diese beschränkt zu sein.

## I Herstellung der Pigmente

### A Klassierung der Substrate

#### Beispiel 1: Klassierung von Glasflakes mit engem Span $\Delta D = 1{,}0$

Eine Suspension von 200 g Glasflakes (GF100M der Firma Glassflake Ltd.) in VE-Wasser (VE = vollentsalzt, ca. 3 Gew.-%ig) wurde über ein 100 µm Sieb klassiert und der Siebdurchgang wiederum über ein 63 µm Sieb gesiebt. Diese Siebprozedur wurde mit auf dem 63 µm Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=50 µm, $D_{50}$=82 µm, $D_{90}$=132 µm, Span $\Delta D = 1{,}0$.

#### Beispiel 2: Klassierung von Glasflakes mit engem Span $\Delta D = 1{,}1$

Eine Suspension von 200 g Glasflakes (GF100M der Firma Glassflake Ltd.) in VE-Wasser (ca. 3 Gew.-%ig) wurde über ein 63 µm Sieb klassiert und der Siebdurchgang wiederum über ein 34 µm Sieb gesiebt. Diese Siebprozedur wurde mit auf dem 34 µm Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=32 µm, $D_{50}$=60 µm, $D_{90}$=100 µm, Span $\Delta D = 1{,}1$.

#### Vergleichsbeispiel 1: Klassierung von Glasflakes mit breitem Span $\Delta D = 2{,}0$

Eine Suspension von 200 g Glasflakes (GF100M der Firma Glassflake Ltd.) in VE-Wasser (ca. 3 Gew.-%ig) wurde über ein 150 µm Sieb klassiert und der Siebdurchgang wiederum über ein 34 µm Sieb gesiebt. Diese Siebprozedur wurde mit auf dem 34 µm Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=25 µm, $D_{50}$=88 µm, $D_{90}$=200 µm, Span ΔD = 2,0.

**Beispiel 3: Klassierung von natürlichem Glimmer mit engem Span ΔD = 1,2**

1 kg Kaliglimmer Mica MD 2800 der Fa. Minelco Specialities Ltd. England wurde 1 h bei 700°C kalziniert, anschließend mit 1000 ml VE-Wasser versetzt und dann in einem Laborkoller der Fa. American Cyanamid Company ca. 1.5 h delaminiert.

Der Kuchen wurde anschließend mit VE-Wasser auf 35 Gew.-% Feststoffgehalt gebracht und über ein Laborsieb des Typs Separator der Fa. Sweco < 250 µm gesiebt.

Die so erhaltene feine Glimmerfraktion wurde dann in einem Labordissolver der Fa. Pendraulik des Typs TD 200 5 h behandelt. Hierbei wurde darauf geachtet, dass durch Kühlung die Temperatur der Suspension 80°C nicht überstieg.

Die Glimmersuspension wurde dann mit VE-Wasser auf 3 Gew.-% Feststoffgehalt verdünnt über ein Laborsieb des Typs Separator der Fa. Sweco < 34µm gesiebt. Der Siebdurchgang wurde dann über ein Sedimentationsgefäß 5 h absedimentiert. Der Überstand wurde abgesaugt, und der Bodensatz erneut mit Wasser aufgenommen, kräftig aufgerührt und erneut 5 h absedimentiert. Dieser Vorgang wurde insgesamt 4 mal wiederholt, bis nahezu kein Überstand mehr zu erkennen war.

Das Sedimentationsgefäß besaß eine zylindrische Form mit den Maßen:

d= 50cm; h= 50cm.
Das Sediment wurde über einen Büchnertrichter abgesaugt und der erhaltene Filterkuchen als Ausgangsmaterial für weitere Beschichtungen verwendet.

Es wurde eine Glimmerfraktion erhalten, die folgende Partikelgrößenverteilung aufwies (MALVERN Mastersizer 2000): $D_{10}$=12,4 µm, $D_{50}$=25,6 µm, $D_{90}$=43,2 µm, Span ΔD = 1,2.

**Beispiel 4: Klassierung von synthetischem Glimmer mit engem Span ΔD = 1,2**

Eine Suspension von 200 g Artificial Mica Sanbao 10-40 (Fa. Shantou F.T.Z. Sanbao Pearl Luster Mica Tech Co., Ltd. China) in VE-Wasser (ca. 3 Gew.-%ig) wurde über ein 34 µm Sieb klassiert und der Siebdurchgang wiederum über ein 20 µm Sieb gesiebt. Diese Siebprozedur wurde mit auf dem 20 µm Sieb erhaltenen Siebrückstand zweimal wiederholt. Es wurde eine Glimmerfraktion erhalten, die folgende Partikelgrößenverteilung aufwies (MALVERN Mastersizer 2000): $D_{10}$=14 µm, $D_{50}$=26 µm, $D_{90}$=45 µm, Span ΔD = 1,2.

**Vergleichsbeispiel 2: Klassierung von natürlichem Glimmer mit breitem Span ΔD = 3,7**

1 kg Kaliglimmer Mica MD 2800 der Fa. Minelco Specialities Ltd. England wurde 1 h bei 700°C kalziniert, anschließend mit 1000 ml VE-Wasser versetzt und dann in einem Laborkoller der Fa. American Cyanamid Company ca. 1 h delaminiert.

Der Kuchen wurde anschließend mit VE-Wasser zu einem Feststoffgehalt von 30 Gew.-% verdünnt und in einem Labordissolver der Fa. Pendraulik des Typs TD 200 1 h behandelt. Hierbei wurde darauf zu geachtet, dass durch Kühlung die Temperatur der Suspension 80°C nicht überstieg.

Anschließend wurde die Suspension mit VE-Wasser auf 2 Gew.-% Feststoffgehalt gebracht und über ein Laborsieb des Typs Separator der Fa. Sweco < 250 µm gesiebt.

Die so erhaltene Glimmerfraktion wurde dann über einen Büchnertrichter abgesaugt und der erhaltene Filterkuchen als Ausgangsmaterial für weitere Beschichtungen verwendet.

Es wurde eine Glimmerfraktion erhalten, die folgende Partikelgrößenverteilung aufwies (MALVERN Mastersizer 2000): $D_{10}$= 19,2 µm, $D_{50}$= 81,7 µm, $D_{90}$= 280,9 µm, Span ΔD = 3,2.

**Vergleichsbeispiel 3: Klassierung von synthetischem Glimmer mit breitem Span ΔD = 3,7**

1 kg handelsüblicher unklassierter synthetischer Mica Artificial Mica Sanbao unclassified (Fa. Shantou F.T.Z. Sanbao Pearl Luster Mica Tech Co., Ltd. China) wurde mit 1000 ml VE-Wasser versetzt und dann in einem Laborkoller der Fa. American Cyanamid Company ca. 1 h delaminiert.

Der Kuchen wurde anschließend mit VE-Wasser zu einem Feststoffgehalt von 25 Gew.-% verdünnt und in einem Labordissolver der Fa. Pendraulik des Typs TD 200 1 h behandelt. Hierbei wurde darauf geachtet, dass durch Kühlung die Temperatur der Suspension 80°C nicht überstieg.

Anschließend wurde die Suspension mit VE-Wasser auf 3 Gew.-% Feststoffgehalt gebracht und über ein Laborsieb des Typs Separator der Fa. Sweco < 250μm gesiebt.

Die so erhaltene Glimmerfraktion wurde dann über einen Büchnertrichter abgesaugt und der erhaltene Filterkuchen als Ausgangsmaterial für weitere Beschichtungen verwendet.

Es wurde eine Glimmerfraktion erhalten, die folgende Partikelgrößenverteilung aufwies (MALVERN Mastersizer 2000): $D_{10}$= 17,7 μm, $D_{50}$= 74,6 μm, $D_{90}$= 292,3 μm, Span $\Delta D$ = 3,7.

## B Herstellung einschichtiger Pigmente (Ausgangsmaterial für Mehrschichtperlglanzpigmente)

### Vergleichsbeispiel 4: Herstellung des Ausgangsmaterials für das Beispiel 5

200 g Glasflakes aus Beispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glasflakes gefällt. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 75 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe erhalten.

### Vergleichsbeispiel 5: Herstellung des Ausgangsmaterials für das Beispiel 6

200 g Glasflakes aus Beispiel 2 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glasflakes gefällt. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 77 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe erhalten.

### Vergleichsbeispiel 6: Herstellung des Ausgangsmaterials für die Beispiele 7 und 8

200 g Glasflakes aus Beispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glasplättchen gefällt. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 3,0 angehoben und sodann eine Lösung von 42 ml $FeCl_3$ (280 g $Fe_2O_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe und leicht orange-roter Absorptionsfarbe erhalten.

### Vergleichsbeispiel 7: Herstellung des Ausgangsmaterials für das Vergleichsbeispiel 9

200 g Glasflakes aus Vergleichsbeispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glasflakes gefällt. Diese Schicht wurde durch Zugabe von einer Lösung, bestehend aus

3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 75 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe erhalten.

**Vergleichsbeispiel 8: natürlicher Glimmer/ $TiO_2$(rutil)**

200 g natürlicher Glimmer aus Beispiel 3 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glimmer gefällt. Diese Schicht wurde durch Zugabe einer Lösung, bestehend aus 5 g $SnCl_4$ x 5 $H_2O$ (in 10 ml konz. HCl plus 50 ml VE-Wasser), unter gleichzeitiger Zudosierung einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 250 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein glänzendes Effektpigment mit silberner Interferenzfarbe erhalten.

**C Herstellung der Mehrschichtperlglanzpigmente**

**Beispiel 5: Glasflakes/ $TiO_2$(rutil)/ $SiO_2$/ $TiO_2$(rutil)**

200 g Glasflakes aus Vergleichsbeispiel 4 wurden in 1300 ml VE-Wasser suspendiert und unter turbulentem Rühren auf 80°C erhitzt. Der pH-Wert wurde mit 5 Gew.-%iger NaOH-Lösung auf 7,5 angehoben und 15 min gerührt. Eine Wasserglaslösung (52 g Wasserglaslösung, 27 Gew.-% $SiO_2$, gemischt mit 52 g VE-Wasser) wurde sodann langsam in die Suspension eingeleitet und der pH-Wert konstant bei pH 7,5 gehalten. Danach wurde noch 20 min nachgerührt und der pH-Wert auf 1,9 gesenkt. Dann schied man eine Schicht "$SnO_2$" auf der $SiO_2$-Oberfläche ab. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 60 ml $TiCl_4$ (200 g $TiO_2$/l VE-Wasser) in die Suspension hinzudosiert. Der pH-Wert wurde dabei durch Gegensteuern mit 10 Gew.-%igen NaOH-Lösung konstant auf pH 1,6 gehalten. Während der Dosierung wurden einzelne Zwischenmuster nach einer Zugabe von 15 ml $TiCl_4$-Lösung (Zwischenmuster 1), 30 ml $TiCl_4$-Lösung (Zwischenmuster 2) und 45 ml $TiCl_4$-Lösung (Zwischenmuster 3) entnommen. Danach wurde noch 15 min nachgerührt, abfiltriert und der Filterkuchen mit VE-Wasser nachgewaschen. Der Filterkuchen und die Zwischenmuster wurden bei 100°C vorgetrocknet und bei 650°C 30 min kalziniert. Es wurden extrem hochglänzende Mehrschichtperlglanzpigmente mit silberner Interferenzfarbe erhalten.

**Vergleichsbeispiel 9: Glasflakes/ $TiO_2$(rutil)/ $SiO_2$/ $TiO_2$(rutil)**

200 g $TiO_2$-beschichtete Glasflakes aus Vergleichsbeispiel 7 wurden in 1300 ml VE-Wasser suspendiert und unter turbulentem Rühren auf 80°C erhitzt. Der pH-Wert wurde mit 5 Gew.-%iger NaOH-Lösung auf 7,5 angehoben und 15 min gerührt. Eine Wasserglaslösung (52 g Wasserglaslösung, 27 Gew.-% $SiO_2$, gemischt mit 52 g VE-Wasser) wurde sodann langsam in die Suspension eingeleitet und der pH-Wert konstant bei pH 7,5 gehalten. Danach wurde noch 20 min nachgerührt und der pH-Wert auf 1,9 gesenkt. Dann schied man eine Schicht "$SnO_2$" auf der $SiO_2$-Oberfläche ab. Diese Schicht wurde durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 60 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Während der Dosierung wurden einzelne Zwischenmuster nach einer Zugabe von 15 ml $TiCl_4$-Lösung (Zwischenmuster 1), 30 ml $TiCl_4$-Lösung (Zwischenmuster 2) und 45 ml $TiCl_4$-Lösung (Zwischenmuster 3) entnommen. Danach wurde noch 15 min nachgerührt, abfiltriert und der Filterkuchen mit VE-Wasser nachgewaschen. Der Filterkuchen und die Zwischenmuster wurden bei 100°C vorgetrocknet und bei 650°C 30 min kalziniert. Es wurden Mehrschichtperlganzpigmente mit silberner Interferenzfarbe erhalten.

**Beispiel 6: Glasflakes/ $TiO_2$(rutil)/ $SiO_2$/ $Fe_2O_3$**

200 g $TiO_2$-beschichtete Glasflakes aus Vergleichsbeispiel 5 wurden in 1300 ml VE-Wasser suspendiert und unter turbulentem Rühren auf 80°C erhitzt. Der pH-Wert wurde mit 5 Gew.-%iger NaOH-Lösung auf 7,5 angehoben und 15 min gerührt. Eine Wasserglaslösung (52 g Wasserglaslösung, 27 Gew.-% $SiO_2$, gemischt mit 52 g VE-Wasser) wurde sodann langsam in die Suspension eingeleitet und der pH-Wert konstant bei pH 7,5 gehalten. Danach wurde noch 20 min nachgerührt, der pH-Wert auf 3,0 gesenkt und sodann eine Lösung von 40 ml $FeCl_3$ (280 g $Fe_2O_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Während der Dosierung wurden einzelne Zwischenmuster nach einer Zugabe von 20 ml $FeCl_3$-Lösung (Zwischenmuster 1) und 30 ml $FeCl_3$-Lösung (Zwischenmuster 2) entnommen. Danach wurde noch 15 min nachgerührt, abilitriert und der Filterkuchen mit VE-Wasser nachgewaschen. Der Filterkuchen und die Zwischenmuster wurden bei 100°C vorgetrocknet und bei 650°C 30 min kalziniert. Es wurden extrem hochglänzende Mehrschichtperlglanzpigmente mit silberner Interferenzfarbe und mit einer leicht orange-rötlichen Absorptionsfarbe erhalten.

**Beispiel 7: Glasflakes/ $Fe_2O_3$/ $SiO_2$/ $Fe_2O_3$**

200 g Glasflakes aus Vergleichsbeispiel 6 wurden in 1400 ml Isopropanol suspendiert und unter turbulentem Rühren auf 70°C erhitzt. Zu dieser Suspension gab man 55 g Tetraethoxysilan, 55 g VE-Wasser und 5 ml 10 Gew.-%ige $NH_3$-Lösung. Die Reaktionsmischung wurde für ca. 12 h gerührt, danach abfiltriert, der Filterkuchen mit Isopropanol nachgewaschen und im Vakuumtrockenschrank bei 100°C getrocknet.

100 g der so erhaltenen $SiO_2$-beschichteten Glasflakes wurden in 700 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den beschichteten Glasflakes gefällt. Diese Schicht wurde durch Zugabe von einer Lösung, bestehend aus 1.5 g $SnCl_4$ x 5 $H_2O$ (in 5 ml konz. HCl plus 25 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 3,0 angehoben und sodann eine Lösung von 17.5 ml $FeCl_3$ (280 g $Fe_2O_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Während der Dosierung wurden einzelne Zwischenmuster nach einer Zugabe von 3.75 ml $FeCl_3$-Lösung (Zwischenmuster 1), 7.5 ml $FeCl_3$-Lösung (Zwischenmuster 2), 10 ml $FeCl_3$-Lösung (Zwischenmuster 3), 12.5 ml $FeCl_3$-Lösung (Zwischenmuster 4) und 15.5 ml $FeCl_3$-Lösung (Zwischenmuster 5) entnommen. Danach wurde noch 15 min nachgerührt, abfiltriert und der Filterkuchen mit VE-Wasser nachgewaschen. Der Filterkuchen und die Zwischenmuster wurden bei 100°C vorgetrocknet und bei 650°C 30 min kalziniert. Es wurden extrem hochglänzende Mehrschichtperlglanzpigmente mit silberner Interferenzfarbe und leicht orange-roter Absorptionsfarbe erhalten.

**Beispiel 8: Glasflakes/ $Fe_2O_3$/ $SiO_2$/ $TiO_2$(rutil)**

200 g Glasflakes aus Vergleichsbeispiel 6 wurden in 1400 ml Isopropanol suspendiert und unter turbulentem Rühren auf 70°C erhitzt. Zu dieser Suspension gab man 55 g Tetraethoxysilan, 55 g VE-Wasser und 5 ml 10 Gew.-%ige $NH_3$-Lösung. Die Reaktionsmischung wurde für ca. 12 h gerührt, danach abfiltriert, der Filterkuchen mit Isopropanol nachgewaschen und im Vakuumtrockenschrank bei 100°C getrocknet.

100 g der so erhaltenen $SiO_2$-beschichteten Glasflakes wurden in 700 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den beschichteten Glasflakes gefällt. Diese Schicht wurde durch Zugabe von einer Lösung, bestehend aus 1.5 g $SnCl_4$ x 5 $H_2O$ (in 5 ml konz. HCl plus 25 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 35 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Während der Dosierung wurden einzelne Zwischenmuster nach einer Zugabe von 7.5 ml $TiCl_4$-Lösung (Zwischenmuster 1), 10 ml $TiCl_4$-Lösung (Zwischenmuster 2), 12.5 ml $TiCl_4$-Lösung (Zwischenmuster 3), 15 ml $TiCl_4$-Lösung (Zwischenmuster 4) und 25 ml $TiCl_4$-Lösung (Zwischenmuster 5) entnommen. Danach wurde noch 15 min nachgerührt, abfiltriert und der Filterkuchen mit VE-Wasser nachgewaschen. Der Filterkuchen und die Zwischenmuster wurden bei 100°C vorgetrocknet und bei 650°C 30 min kalziniert. Es wurden extrem hochglänzende silberfarbene Mehrschichtperlglanzpigmente mit leicht orange-roter Körperfarbe erhalten.

**Vergleichsbeispiel 10: natürlicher Glimmer/ $TiO_2$(rutil)/ $SiO_2$/ $TiO_2$(rutil)**

200 g natürlicher Glimmer aus Vergleichsbeispiel 2 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glimmer gefällt. Diese Schicht wurde durch Zugabe einer Lösung, bestehend aus 5 g $SnCl_4$ x 5 $H_2O$ (in 10 ml konz. HCl plus 50 ml VE-Wasser), unter gleichzeitiger Zudosierung einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 320 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde der pH-Wert mit 5 Gew.-%iger NaOH-Lösung auf 7,5 angehoben und 15 min gerührt. Eine Wasserglaslösung (200 g Wasserglaslösung, 24 Gew.-% $SiO_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 1,9 gesenkt. Dann schied man eine zweite Schicht "$SnO_2$" auf der $SiO_2$-Oberfläche ab. Diese Schicht wurde durch Zugabe von einer Lösung, bestehend aus 5 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 60 ml $TiCl_4$ (200 g $TiO_2$/ l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein schwach glänzendes Mehrschichtperlglanzpigment mit silberner Interferenzfarbe erhalten.

**Vergleichsbeispiel 11: Synthetischer Glimmer/ $Fe_2O_3$/ $SiO_2$/ $TiO_2$(rutil)**

200 g synthetischer Glimmer aus Vergleichsbeispiel 3 wurden in 2000 ml VE-Wasser suspendiert und unter turbulentem Rühren auf 80°C erhitzt. Der pH-Wert der Suspension wurde mit verdünnter HCl auf pH 3,0 eingestellt und sodann eine Lösung von 37,5 ml $FeCl_3$ (280 g $Fe_2O_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Um die Fällung zu vervollständigen wurde die Suspension noch weitere 15 min gerührt. Danach wurde der pH-Wert mit 5 Gew.-%iger NaOH-Lösung auf 7,5 angehoben und 15 min gerührt. Eine Wasserglaslösung (153 g Wasserglaslösung, 20 Gew.-% $SiO_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 1,9 gesenkt. Dann schied man eine Schicht "$SnO_2$" auf der $SiO_2$-Oberfläche ab. Diese Schicht wurde durch Zugabe von einer Lösung, bestehend aus 5 g $SnCl_4$ x 5 $H_2O$ (in 10 ml konz. HCl plus 50 ml VE-Wasser), unter gleichzeitiger Zudosierung einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 150 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein schwach glänzendes, Mehrschichtperlglanzpigment mit silberner Interferenzfarbe und rötlich-brauner Absorptionsfarbe erhalten.

**Beispiel 9: natürlicher Glimmer/ $TiO_2$(rutil)/ $SiO_2$/ $TiO_2$(rutil)**

200 g natürlicher Glimmer aus Beispiel 3 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "$SnO_2$" auf den Glimmer gefällt. Diese Schicht wurde durch Zugabe einer Lösung, bestehend aus 6 g $SnCl_4$ x 5 $H_2O$ (in 10 ml konz. HCl plus 50 ml VE-Wasser), unter gleichzeitiger Zudosierung einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 320 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde der pH-Wert mit 5 Gew.-%iger NaOH-Lösung auf 7,5 angehoben und 15 min gerührt. Eine Wasserglaslösung (200 g Wasserglaslösung, 24 Gew.-% $SiO_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 1,9 gesenkt. Dann schied man eine zweite Schicht "$SnO_2$" auf der $SiO_2$-Oberfläche ab. Diese Schicht wurde durch Zugabe von einer Lösung, bestehend aus 6 g $SnCl_4$ x 5 $H_2O$ (in 10 ml konz. HCl plus 50 ml VE-Wasser), unter gleichzeitiger Zudosierung von einer 10 Gew.-%iger NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 320 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein extrem hochglänzendes Mehrschichtperlglanzpigment mit silberner Interferenzfarbe erhalten.

**Beispiel 10: Synthetischer Glimmer/ $Fe_2O_3$/ $SiO_2$/ $TiO_2$(rutil)**

200 g synthetischer Glimmer aus Beispiel 4 wurden in 2000 ml VE-Wasser suspendiert und unter turbulentem Rühren auf 80°C erhitzt. Der pH-Wert der Suspension wurde mit verdünnter HCl auf pH 3,0 eingestellt und sodann eine Lösung von 60 ml $FeCl_3$ (280 g $Fe_2O_3$/l VE-Wasser) in die Suspension hinzudosiert. Der pH-Wert wurde dabei durch Gegensteuern mit 10 Gew.-%iger NaOH-Lösung konstant auf pH 3,0 gehalten. Um die Fällung zu vervollständigen wurde die Suspension noch weitere 15 min gerührt. Danach wurde der pH-Wert mit 5 Gew.-%iger NaOH-Lösung auf 7,5 angehoben und 15 min gerührt. Eine Wasserglaslösung (185 g Wasserglaslösung, 24 Gew.-% $SiO_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 1,9 gesenkt. Dann schied man eine Schicht "$SnO_2$" auf der $SiO_2$-Oberfläche ab. Diese Schicht wurde durch Zugabe von einer Lösung, bestehend aus 5 g $SnCl_4$ x 5 $H_2O$ (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 200 ml $TiCl_4$ (200 g $TiO_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein extrem hochglänzendes Mehrschichtperlglanzpigment mit silberner Interferenzfarbe und rötlich brauner Absorptionsfarbe erhalten.

**<u>II Physikalische Charakterisierung</u>**

**IIa Winkelabhängige Farbmessungen**

Zur Messung der Chromawerte wurden die Mehrschichtperlglanzpigmente mit einer Pigmentierungshöhe von 6 Gew.-% (bezogen auf das Gesamtgewichtes des Nasslacks) in einen konventionellen Nitrocelluloselack (Dr. Renger Erco Bronzemischlack 2615e; Fa. Morton) eingerührt. Dabei wurden die Mehrschichtperlglanzpigmente vorgelegt und anschließend mit einem Pinsel in den Lack dispergiert.

Der fertige Lack wurde auf einem Rakelabzugsgerät (RK Print Coat Instr. LTd. Citenco Abziehgerät Modell K 101) mit einer Nassfilmdicke je nach $D_{50}$-Wert des Mehrschichtperlglanzpigments gemäß Tabelle 3 auf Byk-Gardner Schwarz-Weiß-Rakelkarten (Byko-Chart 2853) appliziert und nachfolgend bei Raumtemperatur getrocknet.

Mit dem Mehrwinkelfarbmessgerät BYK-MAC (Fa. BYK Gardner) wurden bei einem konstanten Einfallswinkel von 45° (gemäß Herstellerangaben) bei verschiedenen Beobachtungswinkeln relativ zum Glanzwinkel die L*- und C*-Werte bestimmt. Insbesondere waren die Beobachtungswinkel relativ nahe am Glanzwinkel bei 15° sowie -15° relevant. Als relevanter Chromawert der erfindungsgemäßen Mehrschichtperlglanzpigmente wurde der $C^*_{15}$-Wert herangezogen, welcher bei einem 15° vom Glanz entfernten Winkel gemessen wurde.

Stark reflektierende Proben (Idealfall Spiegel) reflektierten nahezu das gesamte eintreffende Licht im sogenannten Glanzwinkel. Hier erschien der Farbeindruck der Interferenzfarbe am stärksten. Je weiter man sich bei der Messung vom Glanzwinkel entfernte, desto weniger Licht und somit Interferenzeffekt konnte gemessen werden.

**IIb Glanzmessungen**

Der Glanz ist ein Maß für die gerichtete Reflexion und lässt sich mittels eines Micro-Tri-Gloss-Gerätes charakterisieren. Stärker streuende Proben weisen mithin einen niedrigen Glanz auf.

Es wurden die Nitrolackapplikationen aus IIa mit Hilfe eines Micro-Tri-Gloss Glanzmessgerätes der Fa. Byk Gardner bei einem Messwinkel von 20° für hochglänzende Proben und bei 60° für mittelglänzende Proben auf schwarzem Hintergrund vermessen. Lagen die Glanzwerte bei 60° über 70 Glanzeinheiten, so werden die Proben bei 20° gemessen (Byk-Gardner Katalog 2007/ 2008, S. 14).

**IIc Partikelgrößenbestimmung:**

Die Größenverteilungskurve wurde mit einem Gerät der Fa. Malvern (Gerät: MALVERN Mastersizer 2000) gemäß Herstellerangaben bestimmt. Hierzu wurden ca 0,1 g des entsprechenden Pigmentes als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Mie-Theorie, welche auch Brechungs- und Absorptionsverhalten der Partikel beinhaltet (Abbildung 3).

Unter der mittleren Größe $D_{50}$ wird im Rahmen dieser Erfindung der $D_{50}$-Wert der Summendurchgangskurve der Volumengemittelten Größenverteilungsfunktion, wie sie durch Laserbeugungsmethoden erhalten werden, verstanden. Der $D_{50}$-Wert gibt an, dass 50 % der Pigmente einen Durchmesser aufweisen, der gleich oder kleiner als der angegebene Wert, beispielsweise 20 $\mu$m, ist.

Entsprechend gibt der $D_{90}$-Wert an, dass 90% der Pigmente einen Durchmesser aufweisen, der gleich oder kleiner des jeweiligen Wertes ist.

Weiterhin gibt der $D_{10}$-Wert an, dass 10% der Pigmente einen Durchmesser aufweisen, der gleich oder kleiner des jeweiligen Wertes ist.

Der Span $\Delta D$, definiert als $\Delta D = (D_{90} - D_{10})/D_{50}$, gibt die Verteilungsbreite wieder.

**III Ergebnisse**

Tabelle 5: Charakterisierung der Effektpigmente

| Effektpigment | Zwischenmuster | Aufbau | Glanz, 20° | $C^*_{15}$ | Span |
|---|---|---|---|---|---|
| Vergleichsbeispiel 4 | | Glas/ $TiO_2$ | 78,0 | 6,0 | 1,1 |
| Vergleichsbeispiel 7 | | Glas/ $TiO_2$ | 67,8 | 6,4 | 2,0 |
| Beispiel 5 | 1 | Glas/$TiO_2$/$SiO_2$/ $TiO_2$ | 70,6 | 12,8 | 1,1 |
| Beispiel 5 | 2 | Glas/$TiO_2$/$SiO_2$/ $TiO_2$ | 86,8 | 15,7 | 1,1 |
| Beispiel 5 | 3 | Glas/$TiO_2$/$SiO_2$/ $TiO_2$ | 94,1 | 16,9 | 1,1 |
| Beispiel 5 | Endprodukt | Glas/$TiO_2$/$SiO_2$/ $TiO_2$ | 109,6 | 13,3 | 1,1 |
| Vergleichsbeispiel 9 | 1 | Glas/$TiO_2$/$SiO_2$/ $TiO_2$ | 60,7 | 11,4 | 2,0 |
| Vergleichsbeispiel 9 | 2 | Glas/$TiO_2$/$SiO_2$/ $TiO_2$ | 71,4 | 13,4 | 2,0 |
| Vergleichsbeispiel 9 | 3 | Glas/$TiO_2$/$SiO_2$/ $TiO_2$ | 74,3 | 12,5 | 2,0 |
| Vergleichsbeispiel 9 | Endprodukt | Glas/$TiO_2$/$SiO_2$/ $TiO_2$ | 77,8 | 14,2 | 2,0 |

Anhand der Daten in Tabelle 5 kann man eindeutig erkennen, dass das erfindungsgemäße Beispiel 5 mit dem Schichtaufbau Glasflake/$TiO_2$/$SiO_2$/$Fe_2O_3$ und geringem Span $\Delta D$ = 1,1 einen starken Glanzgewinn bei steigender Titandioxidschichtdicke aufwies. Der Glanzgewinn im Vergleich zum Ausgangsmaterial Vergleichsbeispiel 4 betrug hierbei 31.6 Einheiten. Auch für das Vergleichsbeispiel 9 mit großem Span $\Delta D$ = 2,0 fand man zwar erhöhte Glanzwerte, aber die Steigerung betrug lediglich 10 Glanzeinheiten (Abbildung 4).

Eine Erhöhung der $TiO_2$-Schichtdicke in den Vergleichsbeispielen 4 bzw. 7 würde zu farbigen, nichtsilbernen Interferenzeffekten führen.

Ähnliche Effekte fand man auch für das erfindungsgemäße Beispiel 6 (Tabelle 6), welches einen Schichtaufbau Glasflake/$TiO_2$/$SiO_2$/$Fe_2O_3$ besitzt, wobei hier der Glanzwert um 17.4 Einheiten gesteigert werden konnte (Abbildung 5). Eine Erhöhung der $TiO_2$-Schichtdicke im Vergleichsbeispiel 5 würde zu farbigen, nichtsilbernen Interferenzeffekten führen.

Tabelle 6: Charakterisierung der Effektpigmente

| Effektpigment | Zwischenmuster | Aufbau | Glanz, 20° | $C^*_{15}$ | Span |
|---|---|---|---|---|---|
| Vergleichsbeispiel 5 | | Glas/$TiO_2$ | 44,0 | 5,7 | 1,1 |
| Beispiel 6 | 1 | Glas/$TiO_2$/$SiO_2$/$Fe_2O_3$ | 51,7 | 5,9 | 1,1 |

(fortgesetzt)

| Effektpigment | Zwischenmuster | Aufbau | Glanz, 20° | $C^*_{15}$ | Span |
|---|---|---|---|---|---|
| Beispiel 6 | 2 | Glas/$TiO_2$/$SiO_2$/$Fe_2O_3$ | 59,9 | 3,9 | 1,1 |
| Beispiel 6 | Endprodukt | Glas/$TiO_2$/$SiO_2$/$Fe_2O_3$ | 61,4 | 4,9 | 1,1 |

Auch die erfindungsgemäßen Beispiele 7 und 8 (Tabelle 7), bei denen Glasflakes mit engem Span zuerst mit einem absorbierenden Eisenoxid beschichtet wurden (Beispiel 7) und anschließend mit $SiO_2$ und $Fe_2O_3$ (Beispiel 6) bzw. $SiO_2$ und $TiO_2$ (Beispiel 8) zeigten deutliche Glanzgewinne. So fand man für Beispiel 7-Endprodukt einen Glanzgewinn von 14.9 Einheiten und für Beispiel 8-Endprodukt einen Glanzgewinn von 17.1 Einheiten (Abbildung 6).

Eine Erhöhung der $Fe_2O_3$-Schichtdicke im Vergleichsbeispiel 6 würde zu farbigen, nichtsilbernen Interferenzeffekten führen.

Tabelle 7: Charakterisierung der Effektpigmente

| Effektpigment | Zwischenmuster | Aufbau | Glanz, 20° | $C^*_{15}$ | Span |
|---|---|---|---|---|---|
| Vergleichsbeispiel 6 | | Glas/$Fe_2O_3$ | 62,9 | 4,1 | 1,1 |
| Beispiel 7 | 1 | Glas/ $Fe_2O_3$/$SiO_2$/$Fe_2O_3$ | 53,2 | 1,5 | 1,1 |
| Beispiel 7 | 2 | Glas/ $Fe_2O_3$/$SiO_2$/$Fe_2O_3$ | 54,9 | 2,2 | 1,1 |
| Beispiel 7 | 3 | Glas/ $Fe_2O_3$/$SiO_2$/$Fe_2O_3$ | 63,6 | 2,4 | 1,1 |
| Beispiel 7 | 4 | Glas/ $Fe_2O_3$/$SiO_2$/$Fe_2O_3$ | 68,4 | 2,6 | 1,1 |
| Beispiel 7 | 5 | Glas/ $Fe_2O_3$/$SiO_2$/$Fe_2O_3$ | 71,0 | 3,2 | 1,1 |
| Beispiel 7 | Endprodukt | Glas/ $Fe_2O_3$/$SiO_2$/$Fe_2O_3$ | 77,8 | 4,2 | 1,1 |
| Beispiel 8 | 1 | Glas/ $Fe_2O_3$/$SiO_2$/$TiO_2$ | 55,3 | 2,8 | 1,1 |
| Beispiel 8 | 2 | Glas/ $Fe_2O_3$/$SiO_2$/$TiO_2$ | 60,1 | 3,0 | 1,1 |
| Beispiel 8 | 3 | Glas/ $Fe_2O_3$/$SiO_2$/$TiO_2$ | 60,7 | 3,9 | 1,1 |
| Beispiel 8 | 4 | Glas/ $Fe_2O_3$/$SiO_2$/$TiO_2$ | 63,8 | 3,8 | 1,1 |
| Beispiel 8 | 5 | Glas/ $Fe_2O_3$/$SiO_2$/$TiO_2$ | 69,0 | 4,1 | 1,1 |
| Beispiel 8 | Endprodukt | Glas/ $Fe_2O_3$/$SiO_2$/$TiO_2$ | 80,0 | 3,0 | 1,1 |

Auch bei den Glimmer-basierenden erfindungsgemäßen Beispielen 9 und 10 ist ein extremer Glanzsteigerungseffekt besonders zu den Vergleichsbeispielen 10 und 11 bei gleichem Schichtaufbau aufgrund des engen Spans zu beobachten (Tabelle 8). Weiterhin zeigt sich auch hier die zusätzliche Glanzsteigerung aufgrund der Mehrschichttechnologie [Vergleich Beispiel 9 (Aufbau: Glimmer/$TiO_2$/$SiO_2$/$TiO_2$) zu Vergleichsbeispiel 8 (Aufbau: Glimmer/$TiO_2$)].

**Tabelle 8:** Charakterisierung der Effektpigmente

| Effektpigment | Aufbau | Glanz, 60° | $C^*_{15}$ | Span |
|---|---|---|---|---|
| Beispiel 9 | nat. Glimmer/$TiO_2$/$SiO_2$/$TiO_2$ | 75,1 | 5,9 | 1,2 |
| Beispiel 10 | synth. Glimmer/$Fe_2O_3$/$SiO_2$/$TiO_2$ | 58,2 | 16,2 | 1,2 |
| Vergleichsbeispiel 8 | nat. Glimmer/$TiO_2$ | 69,0 | 8,9 | 1,2 |
| Vergleichsbeispiel 10 | nat. Glimmer/$TiO_2$/$SiO_2$/$TiO_2$ | 18,6 | 16,8 | 3,7 |
| Vergleichsbeispiel 11 | synth. Glimmer/$Fe_2O_3$/$SiO_2$/$TiO_2$ | 20,1 | 9,7 | 3,7 |

## IV. Anwendungstechnische Beispiele

In den nachfolgenden kosmetischen Anwendungsbeispielen wurden die erfindungsgemäßen Mehrschichtperlglanzpigmente verwendet, die nach einem der vorstehenden Beispiele hergestellt wurden.

Beispiel 11: Body Lotion Water-in-Silicone

| INCI Name | Produktname | Gew-% | Lieferant |
|---|---|---|---|
| *Phase* A | | *100.00* | |
| Cyclopentasiloxane (and) Dimethiconol | Dow Corning 1501 | 11.20 | www.dowcorning.com |
| Cyclopentasiloxane | Dow Corning 245 | 5.75 | www.dowcorning.com |
| Cyclopentasiloxane (and) PEG/PPG/-18/18 Dimethicone | Dow Corning 5225 C | 13.80 | www.dowcorning.com |
| C 30-45 Alkyl Methicone | Dow Corning Cosmetic Wax AMS-C30 | 3.45 | www.dowcorning.com |
| **Mehrschichtperlglanzpigment** | **Mehrschichtperlglanzpigment** | **1.00** | |
| *Phase B* | | | |
| Polysorbate 20 | Tween 20 | 0.60 | www.uniqema.com |
| Phenoxyethanol (and) Methylparaben (and) Ethylparaben (and) Butylparaben | Uniphen P-23 | 0.35 | www.induchem.com |
| Sodium Chloride | Sodium Chloride | 0.75 | www.vwr.com |
| Aqua | Wasser | 63.10 | |

Das Mehrschichtperlglanzpigment kann in einem Bereich von 0,5 bis 2,5 Gew.-% eingesetzt werden. Der Ausgleich kann mit Wasser erfolgen.

Phase A wurde gemischt und auf 75°C erhitzt, Phase B nach dem Mischen auf 70°C erhitzt, anschließend wurde Phase B langsam unter Homogenisierung zu Phase A hinzugefügt. Unter Rühren wurde die Emulsion abgekühlt und in ein angemessenes Behältnis abgefüllt.

Beispiel 12: Körperpuder

| INCI Name | Produktname | Gew-% | Lieferant |
|---|---|---|---|
| *Phase A* | | *100.00* | |
| Mica | Silk Mica | 40.70 | www.vwr.com |
| Talc | Talc Powder | 18.00 | www.riedeldehaen.com |
| Boron Nitride | Softouch CCS 102 | 5.00 | www.advceramics.com |
| Nylon-12 | Orgasol 2002 D/Nat | 8.00 | www.atofinachemicals.com |
| Magnesium Stearate | Magnesium Stearate | 6.00 | www.sigmaaldrich.com |
| Methylparaben, Propylparaben | Rokonsal SSH-1 | 0.30 | www.biochema.com |
| Mica (and) Iron Oxides | Prestige Soft Bronze | 9.00 | www.eckart.net |
| **Mehrschichtperlglanzpigment** | **Mehrschichtperl glanzpigment** | **2.00** | |
| Mica (and) Titanium Dioxide | Prestige Magic Orange | 9.00 | www.eckart.net |

(fortgesetzt)

| | | | |
|---|---|---|---|
| *Phase B* | | | |
| Tridecyl Stearate (and) Tridecyl Trimellitate (and) Dipentaerythrityl Hexacaprylate/Hexacaprate | Lipovol MOS-130 | 2.00 | www.lipochemicals.com |

Das Mehrschichtperlglanzpigment kann in einem Bereich von 0,2 bis 5,0 Gew.-% eingesetzt werden. Der Ausgleich kann mit Mica erfolgen.

Die Inhaltsstoffe der Phase A wurden zusammengemischt, anschließend wurde Phase B zu Phase A zugegeben. Nach Vermischen in ein Gefäß abfüllen.

Beispiel 13: Creme Lidschatten

| **INCI Name** | **Produktname** | **Gew-%** | **Lieferant** |
|---|---|---|---|
| *Phase A* | | 100.00 | |
| Castor Oil | Castor Oil | 31.70 | www.riedeldehaen.com |
| Octyl Palmitate | Liponate EHP | 6.00 | www.lipochemicals.com |
| Cocos Nucifera (Coconut) Oil | Lipovol C-76 | 7.00 | www.lipochemicals.com |
| Bees Wax | Ewacera 12 | 6.00 | www.wagnerlanolin.com |
| Isopropyl Lanolate | Ewalan IP | 5.00 | www.wagnerlanolin.com |
| Persea Gratissima (Avocado) Oil and Hydrogenated Avocado Oil | Avocado Butter | 7.00 | www.impag.de |
| Magnesium Stearate | Magnesium Stearate | 3.00 | www.sigmaaldrich.com |
| Bis-Hydroxyethoxypropyl Dimethicone | Dow Corning 5562 Carbinol Fluid | 7.00 | www.dowcorning.com |
| Dimethicone/ Vinyl Dimethicone Crosspolymer and Silica | Dow Corning 9701 Cosmetic Powder | 5.00 | www.dowcoming.com |
| Phenoxyethanol (and) Methylparaben (and) Ethylparaben (and) Butylparaben | Uniphen P-23 | 0.30 | www.induchem.com |
| *Phase B* | | | |
| Mica (and) Iron Oxides | Prestige Soft Bronze | 21.00 | www.eckart.net |
| **Mehrschichtperlglanzpigment** | **Mehrschichtperlglanzpigment** | **1.00** | |

Das Mehrschichtperlglanzpigment kann in einem Bereich von 0,1 bis 5,0 Gew.-% eingesetzt werden. Der Ausgleich kann mit Castor Oil erfolgen.

Phase A wurde gemischt und auf 85°C erhitzt, Inhaltsstoffe von Phase B wurden ebenfalls zusammengemischt und anschließend unter Rühren zu Phase A hinzugegeben. Nach Abfüllen in ein entsprechendes Behältnis wurde die Mischung auf Raumtemperatur abgekühlt.

Beispiel 14: Foundation

| INCI Name | Produktname | Gew-% | Lieferant |
|---|---|---|---|
| *Phase A* | | 100.00 | |
| Hydrogenated Polydecene | Ritadecene 20 | 9.00 | www.ritacorp.com |
| Caprylic/ Capric Triglyceride | Liponate GC-K | 5.00 | www.lipochemicals.com |
| Prunus Amygdalus Dulcis (Sweet Almond) Oil | Sweet Almond Oil | 4.00 | www.jandekker.com |
| Caprylyl Trimethicone | SilCare Silicone 31M50 | 4.00 | www.clariant.com |
| Caprylyl Methicone | SilCare Silicone 41M15 | 3.00 | www.clariant.com |
| Steareth-2 | Volpo S2 | 1.60 | www.croda.com |
| Steareth-20 | Sympatens AS/200 G | 2.40 | www.kolb.ch |
| *Phase B* | | | |
| Talc | Talc Powder | 4.50 | www.vwr.com |
| Mica (and) Iron Oxides | Prestige Soft Beige | 4.00 | www.eckart.net |
| Mica (and) Titanium Dioxide | Prestige Soft Silver | 1.00 | www.eckart.net |
| **Mehrschichtperlglanzpigment** | **Mehrschichtperlglanzpigment** | **0.50** | |
| *Phase C* | | | |
| Glycerin | Pricerine 9090 | 5.00 | www.brenntag.com |
| Aqua | Wasser | 55.20 | |
| Ammonium Acryloyldimehtyltauratel VP Copolymer | Aristoflex AVC | 0.40 | www.simon-und-werner.com |
| *Phase D* | | | |
| Propylene Glycol (and) Diazolidinyl Urea (and) Methylparaben (and) Propylparaben | Nipaguard PDU | 0.40 | www.simon-und-werner.com |

Das Mehrschichtperlglanzpigment kann in einem Bereich von 0,1 bis 1,0 Gew.-% eingesetzt werden. Der Ausgleich kann mit Wasser erfolgen.

Phase A und Phase B wurden separat eingewogen. Phase A wurde unter Rühren auf 70°C erhitzt und Phase B unter Rühren hinzugefügt. Phase C wurde gut gemischt bis Aristoflex aufgelöst war und anschließend ebenfalls auf 70°C erhitzt. Phase C wurde zu Phase AB hinzugefügt und nach Abkühlen auf 40°C wurde Phase D hinzugefügt.

Beispiel 15: Haargel

| INCI Name | Produktname | Gew-% | Lieferant |
|---|---|---|---|
| *Phase A* | | 100.00 | |
| **Mehrschichtperl-glanzpigment** | **Mehrschichtperlglanzpigment** | 0.10 | |
| Ammonium Acryloyldimehtyltaurate/ VP Copolymer | Aristoflex AVC | 1.40 | www.clariant.com |
| Citric Acid | Citric Acid | 0.10 | www.vwr.com |
| Aqua | Wasser | 55.10 | |

(fortgesetzt)

| | | | |
|---|---|---|---|
| *Phase B* | | | |
| PVP | Luviskol K 30 Powder | 1.50 | www.basf.com |
| Propylene Glycol, Diazolidinyl, Urea, Methylparaben, Propylparaben | Germaben II | 0.20 | www.ispcorp.com |
| Triethanolamine | Triethanolamine | 1.20 | www.vwr.com |
| Water | Aqua | 40.40 | |

Das Mehrschichtperlglanzpigment kann in einem Bereich von 0,01 bis 0,5 Gew.-% eingesetzt werden. Der Ausgleich kann mit Wasser erfolgen.

Das Mehrschichtperlglanzpigment wurde mit dem Wasser von Phase A verrührt, Aristoflex AVP und Citric Acid wurde unter Rühren hinzugegeben und bei einer Geschwindigkeit von 800 rpm für 15 Minuten gemischt. Die Inhaltsstoffe von Phase B wurden aufgelöst bis eine homogene Lösung entstand, anschließend wurde Phase B zu Phase A gegeben und gemischt.

Beispiel 16: Hair Mascara

| **INCI Name** | **Produktname** | **Gew-%** | **Lieferant** |
|---|---|---|---|
| *Phase A* | | *100.00* | |
| Polyquaternium-16 | Luviquat FC 905 (Luviquat Exellence) | 2.70 | www.basf.com |
| Propylene Glycol | 1,2-Propanediol | 1.80 | www.vwr.com |
| Methylparaben | Methyl-4-hydroxybenzoate | 0.20 | www.sigmaaldrich.com |
| Aqua | Wasser | 64.45 | |
| *Phase B* | | | |
| Cetearyl Alcohol | Lanette O | 5.00 | www.cognis.com |
| Dimethicone | Dow Corning 200 Fluid/ 350 cst | 1.00 | www.dowcorning.com |
| Ceteareth-25 | Cremophor A 25 | 2.00 | www.basf.com |
| Propylparaben | Propyl-4-hydroxybenzoat | 0.10 | www.sigmaaldrich.com |
| *Phase C* | | | |
| Hydroxypropylcellulose | Klucel G | 0.50 | www.herc.com |
| Magnesium Aluminium Silicate | Veegum HV | 0.50 | www.rtvanderbilt.com |
| Aqua | Wasser | 19.00 | |
| *Phase D* | | | |
| **Mehrschichtperlglanzpigment** | **Mehrschichtperlglanzpigment** | **2.50** | |
| Phenoxyethanol (and) Methylparaben (and) Butylparaben (and) Ethylparaben (and) Propylparaben (and) Isobutylparaben | Phenonip | 0.20 | www.clariant.com |

(fortgesetzt)

| | | | |
|---|---|---|---|
| *Phase D* | | | |
| Fragrance | Blue Shadow ÖKO | 0.05 | www.bell-europe.com |

Das Mehrschichtperlglanzpigment kann in einem Bereich von 0,5 bis 5,0 Gew.-% eingesetzt werden. Der Ausgleich kann mit Wasser von Phase A erfolgen.

Phase A und Phase B wurden getrennt auf 80°C erhitzt, danach wurde Phase B langsam zu Phase A hinzugefügt. In einem separaten Gefäß wurden Klucel und Veegum dem Wasser von Phase C hinzugegeben. Anschließend wurde Phase AB auf 40°C abgekühlt und während dem Abkühlen Phase C und D unter rühren hinzugemischt.

Beispiel 17: Lip Gloss

| **INCI Name** | **Produktname** | **Gew-%** | **Lieferant** |
|---|---|---|---|
| *Phase A* | | *100.00* | |
| Hydrogenated Polyisobutene (and) Ethylene/Propylene/Styrene Copolymer (and) Butylene/Ethylene/Styrene Copolymer | Versagel ME 750 | 79.00 | www.penreco. com |
| Simmondsia Chinensis (Jojoba) Seed Oil | Jojoba Oil - Natural/Golden | 2.00 | www.biochemica.com |
| Caprylyl Trimethicone | Silcare Silicone 31M50 | 7.00 | www.clariant.com |
| Stearyl Dimethicone | Silcare Silicone 41 M65 | 3.20 | www.clariant.com |
| Hydrogenated Polydecene | Nexbase 2002 | 4.00 | www.jandekker. com |
| Isopropyl Myristate | Isopropyl Myristate | 4.50 | www.vwr.com |
| *Phase B* | | | |
| **Mehrschichtperlglanzpigment** | **Mehrschichtperlglanzpigment** | **0.10** | |
| Propylparaben | Propyl-4-hydroxybenzoat | 0.20 | www. sigmaaldrich.com |

Das Mehrschichtperlglanzpigment kann in einem Bereich von 0,01 bis 0,50 Gew.-% eingesetzt werden. Der Ausgleich kann mit Versagel ME 750 erfolgen.

Phase A wurde auf 85°C erhitzt, anschließend wurden die Inhaltstoffe der Phase B einzeln zu Phase A hinzugegeben und gerührt bis eine gleichmäßige Konsistenz entstand und dann in ein Lip Gloss Gefäß abgefüllt.

Beispiel 18: Lip Liner

| **INCI Name** | **Produktname** | **Gew-%** | **Lieferant** |
|---|---|---|---|
| *Phase A* | | *100.00* | |
| Hydrogenated Coco-Glycerides | Softisan 100 | 12.35 | www.sasolwax.com |
| Candelilla Wax | Ewacera 42 | 14.00 | www.wagnerlanolin.de |
| Magnesium Stearate | Magnesium Stearate | 6.00 | www.sigmaaldrich.com |
| Stearic Acid | Kortacid 1895 | 8.50 | www.akzonobel.com |
| Hydrogenated Coconut Oil | Lipex 401 | 8.00 | www.karlshamns.com |
| Cetyl Palmitate | Walrath synthetic | 7.00 | www.kahlwax.de |
| Caprylic/ Capric Triglyceride | Liponate GC-K | 3.60 | www.lipochemicals.com |

(fortgesetzt)

| INCI Name | Produktname | Gew-% | Lieferant |
|---|---|---|---|
| *Phase A* | | *100.00* | |
| Soybean Glycerides (and) Butyrospermum Parkii | Lipex L'sens | 15.00 | www.karlshamns.com |
| Tocopheryl Acetate | D,L-Alpha-Tocopherolacetat | 0.25 | www.dsm.com |
| Methylparaben; Propylparaben | Rokonsal SSH-1 | 0.30 | www.biochema.com |
| *Phase B* | | | |
| Mica (and) Titanium Dioxide (and) Ferric Ferrocyanide | Prestige Sapphire | 7.50 | www.eckart.net |
| Mica (and) Iron Oxides | Prestige Copper | 7.50 | www.eckart.net |
| Mica (and) Titanium Dioxide | Prestige Soft Silver | 5.00 | www.eckart.net |
| **Mehrschichtperlglanzpigment** | **Mehrschichtperlglanzpigment** | **5.00** | |

Das Mehrschichtperlglanzpigment kann in einem Bereich von 0,5 bis 10 Gew.-% eingesetzt werden. Der Ausgleich kann mit anderen Pigmenten erfolgen, die Pigmentierung muss bei 25 Gew.-% gehalten werden.

Phase A wurde auf 85°C erhitzt und anschließend die Phase B der Phase A unter Rühren hinzugefügt bis sich eine gleichförmige Masse ergab. Danach wurde die Mischung heiß in eine Stiftform eingefüllt.

Beispiel 19: Lippenstift

| INCI Name | Produktname | Gew-% | Lieferant |
|---|---|---|---|
| *Phase A* | | *100.00* | |
| Carnauba Wax | Ewacera 34 | 4.50 | www.wagnerlanolin.de |
| Bees Wax | Ewacera 12 | 3.50 | www.wagnerlanolin.de |
| Candelilla Wax | Ewacera 42 | 4.00 | www.wagnerlanolin.de |
| Microcrystalline Wax | Parcera MW | 7.20 | www.paramelt.com |
| Cetyl Palmitate | Walrath synthetic | 2.00 | www.kahlwax.de |
| Hydrogenated Coco-Glycerides | Softisan 100 | 5.00 | www.sasolwax.com |
| Petrolatum | Penreco Blond | 5.80 | www.penreco.com |
| Cetearyl Octanoate | Luvitol EHO | 10.70 | www.basf.com |
| Tocopheryl Acetate | D,L-Alpha-Tocopherolacetat | 0.50 | www.dsm.com |
| Castor Oil | Castor Oil | 36.60 | www.riedeldehaen.com |
| *Phase B* | | | |
| Mica (and) Iron Oxide | Prestige Fire-red | 16.00 | www.eckart.net |
| **Mehrschichtperlglanzpigment** | **Mehrschichtperlglanzpigment** | **5.00** | |
| Methylparaben, Propylparaben | Rokonsal SSH-1 | 0.20 | www.biochema.com |

Das Mehrschichtperlglanzpigment kann in einem Bereich von 0,5 bis 10,0 Gew.-% eingesetzt werden. Der Ausgleich kann mit anderen Pigmenten erfolgen, die Pigmentierung muss bei 21 Gew.-% gehalten werden.

Phase A wurde auf 85°C erhitzt, danach wurde Phase B der Phase A hinzugegeben und gemischt. Anschließend wurde die Mischung mit einer Temperatur von 75°C in einem Lippenstiftform abgefüllt.

Beispiel 20: Liquid Eyeliner

| INCI Name | Produktname | Gew-% | Lieferant |
|---|---|---|---|
| *Phase A* | | *100.00* | |
| Aqua | Wasser | 64.70 | |
| Water/ carbon black dispersion | MBD 201 | 3.00 | www.geotech.nl |
| Acrylates Copolymer | Covacryl E14 | 10.00 | www.lcw.fr |
| Magnesium Aluminium Silicate | Veegum HV | 1.00 | www.cherbsloeh.de |
| *Phase B* | | | |
| Propylene Glycol | 1,2 Propandiol | 3.00 | www.vwr.com |
| Triethanolamine | Triethanolamine | 1.40 | www.vwr.com |
| *Phase C* | | | |
| Xanthan Gum | Keltrol T | 0.30 | www.cpkelco.com |
| *Phase D* | | | |
| **Mehrschichtperlglanzpigment** | **Mehrschichtperlglanzpigment** | **5.00** | |
| Mica | Silk Mica | 2.00 | www.vwr.com |
| *Phase E* | | | |
| Stearic Acid | Kortacid 1895 | 2.80 | www.akzonobel.de |
| Glyceryl Stearate | Aldo MS K FG | 0.80 | www.lonza.com |
| Oleyl Alcohol | HD-Ocenol 90/95 V | 0.50 | www.biesterfeld.com |
| Phenoxyethanol (and) Methylparaben (and) Ethylparaben (and) Butylparaben | Uniphen P-23 | 0.50 | www.induchem.com |
| *Phase F* | | | |
| Dimethicone (and) Trisiloxane | Dow Corning 2-1184 Fluid | 5.00 | www.dowchemicals. com |

Das Mehrschichtperlglanzpigment kann in einem Bereich von 0,5 bis 8,0 Gew.-% eingesetzt werden. Der Ausgleich kann mit Wasser erfolgen.

Veegum wurde in Phase A dispergiert und 15 Minuten gerührt, danach wurde Phase B zu Phase A hinzugefügt, anschließend Phase C zu Phase AB und erneut 10 Minuten verrührt. Anschließend wurde Phase D zu Phase ABC hinzugefügt und auf 75°C erhitzt, Phase E wurde ebenfalls auf 75°C erwärmt und danach zu Phase ABCD hinzugefügt. Nach dem Abkühlen auf 60°C wurde Phase F zugefügt und in ein geeignetes Gefäß abgefüllt.

## Patentansprüche

**1.** Mehrschichtperlglanzpigmente mit silberner Interferenzfarbe basierend auf plättchenförmigen transparenten Substraten mit optisch wirksamer Beschichtung,
**dadurch gekennzeichnet,**
**dass** die optisch wirksame Beschichtung wenigstens

a) eine hochbrechende Schicht A mit einem Brechungsindex $n \geq 1{,}8$
b) eine niedrigbrechende Schicht B mit einem Brechungsindex $n < 1{,}8$
c) eine hochbrechende Schicht C mit einem Brechungsindex $n \geq 1{,}8$ sowie
d) optional eine äußere Schutzschicht D

umfasst und dass die Mehrschichtperlglanzpigmente die Kennzahlen $D_{10}$, $D_{50}$, $D_{90}$ aus der Summenhäufigkeits-

verteilung der volumengemittelten Größenverteilungsfunktion mit einem Span $\Delta D$ von 0,7 - 1,4 aufweisen, wobei der Span $\Delta D$ gemäß der Formel $\Delta D = (D_{90} - D_{10})/ D_{50}$ berechnet ist.

**2.** Mehrschichtperlglanzpigmente mit silberner Interferenzfarbe nach Anspruch 1,
**dadurch gekennzeichnet,**
**dass** Schicht A und/ oder Schicht C unabhängig voneinander selektiv und/ oder nichtselektiv absorbierend oder nicht absorbierend sind.

**3.** Mehrschichtperlglanzpigmente mit silberner Interferenzfarbe nach einem der vorstehenden Ansprüche,
**dadurch gekennzeichnet,**
**dass** Schicht B nicht absorbierend ist.

**4.** Mehrschichtperlglanzpigmente mit silberner Interferenzfarbe nach einem der vorstehenden Ansprüche,
**dadurch gekennzeichnet,**
**dass** die Mehrschichtperlglanzpigmente einen Span $\Delta D$ von 0,7 bis 1,3 aufweisen.

**5.** Mehrschichtperlglanzpigmente mit silberner Interferenzfarbe nach einem der vorstehenden Ansprüche,
**dadurch gekennzeichnet,**
**dass** die optische Schichtdicke von Schicht A und Schicht C jeweils im Bereich von 50 bis 200 nm liegt.

**6.** Mehrschichtperlglanzpigmente mit silberner Interferenzfarbe nach einem der vorstehenden Ansprüche,
**dadurch gekennzeichnet,**
**dass** die optische Schichtdicke der Schicht B in einem Bereich von 30 bis 150 nm liegt.

**7.** Mehrschichtperlglanzpigmente mit silberner Interferenzfarbe nach einem der vorstehenden Ansprüche,
**dadurch gekennzeichnet,**
**dass** die Schicht A und/ oder C Titandioxid und/ oder Eisenoxid umfasst.

**8.** Mehrschichtperlglanzpigmente mit silberner Interferenzfarbe nach einem der vorstehenden Ansprüche,
**dadurch gekennzeichnet,**
**dass** die Schicht B Siliziumdioxid umfasst.

**9.** Mehrschichtperlglanzpigmente mit silberner Interferenzfarbe nach einem der vorstehenden Ansprüche,
**dadurch gekennzeichnet,**
**dass** die plättchenförmigen transparenten Substrate der Mehrschichtperlglanzpigmente mit silberner Interferenzfarbe aus der Gruppe, bestehend aus natürlichem Glimmer, synthetischem Glimmer, Glasflakes, $SiO_2$-Plättchen, $Al_2O_3$-Plättchen und deren Gemische, ausgewählt sind.

**10.** Mehrschichtperlglanzpigmente mit silberner Interferenzfarbe nach einem der vorstehenden Ansprüche,
**dadurch gekennzeichnet,**
**dass** die Mehrschichtperlglanzpigmente einen Chromawert $\mathbf{C^*_{15}}$ von $\leq 20$ aufweisen.

**11.** Verfahren zur Herstellung der Mehrschichtperlglanzpigmente mit silberner Interferenzfarbe nach einem der vorstehenden Ansprüche,
**dadurch gekennzeichnet,**
**dass** es folgende Schritte umfasst:

i) Größenklassieren der zu beschichtenden plättchenförmigen transparenten Substrate
ii) Beschichten der plättchenförmigen transparenten Substrate mit wenigstens in Anspruch 1 genannten Schichten A bis C sowie optional Schicht D.

**12.** Verwendung der Mehrschichtperlglanzpigmente mit silberner Interferenzfarbe nach einem der Ansprüche 1 bis 10 in kosmetischen Formulierungen, Kunststoffen, Folien, Textilien, keramischen Materialien, Gläsern und Beschichtungszusammensetzungen wie Farben, Druckfarben, Tinten, Lacken oder Pulverlacken.

**13.** Zubereitung, die die Mehrschichtperlglanzpigmente nach einem der Ansprüche 1 bis 10 enthält.

**14.** Gegenstand, der mit Mehrschichtperlglanzpigmenten nach einem der Ansprüche 1 bis 10 beschichtet ist.

## Claims

1. Multi-layered pearlescent pigments having a silver interference colour based on platelet-shaped transparent substrates with an optically active coating,
**characterised in that**
the optically active coating comprises at least

   a) a high index layer with a refractive index $n \geq 1.8$,
   b) a low index layer B with a refractive index $n < 1.8$,
   c) a high index layer C with a refractive index $n \geq 1.8$ and
   d) optionally an outer protective layer D,

   and the multi-layered pearlescent pigments have the indices $D_{10}$, $D_{50}$, $D_{90}$ from the cumulative frequency distribution of the volume-averaged size distribution function with a span $\Delta D$ of 0.7 - 1.4, and the span $\Delta D$ is calculated by means of the formula $\Delta D = (D_{90} - D_{10})/ D_{50}$.

2. Multi-layered pearlescent pigments having a silver interference colour as claimed in claim 1,
**characterised in that**
layer A and/or layer C are, independently of one another, selectively and/or non-selectively absorbing or non-absorbing.

3. Multi-layered pearlescent pigments having a silver interference colour as claimed in one of the preceding claims,
**characterised in that**
layer B is non-absorbing.

4. Multi-layered pearlescent pigments having a silver interference colour as claimed in one of the preceding claims,
**characterised in that**
the multi-layered pearlescent pigments have a span $\Delta D$ of 0.7 to 1.3.

5. Multi-layered pearlescent pigments having a silver interference colour as claimed in one of the preceding claims,
**characterised in that**
the optical layer thickness of layer A and layer C is respectively within the range of 50 to 200 nm.

6. Multi-layered pearlescent pigments having a silver interference colour as claimed in one of the preceding claims,
**characterised in that**
the optical layer thickness of layer B is within a range of 30 to 150 nm.

7. Multi-layered pearlescent pigments having a silver interference colour as claimed in one of the preceding claims,
**characterised in that**
layer A and/or C comprises titanium dioxide and/or iron oxide.

8. Multi-layered pearlescent pigments having a silver interference colour as claimed in one of the preceding claims,
**characterised in that**
layer B comprises silicon dioxide.

9. Multi-layered pearlescent pigments having a silver interference colour as claimed in one of the preceding claims,
**characterised in that**
the platelet-shaped transparent substrates of the multi-layered pearlescent pigments having a silver interference colour are selected from the group comprising natural mica, synthetic mica, glass flakes, $SiO_2$ platelets, $Al_2O_3$ platelets and mixtures thereof.

10. Multi-layered pearlescent pigments having a silver interference colour as claimed in one of the preceding claims,
**characterised in that**
the multi-layered pearlescent pigments have a chroma $C^*_{15}$ of $\geq 20$.

11. Method of producing multi-layered pearlescent pigments having a silver interference colour as claimed in one of the preceding claims, **characterised in that**
it comprises the following steps:

i) size classifying the platelet-shaped transparent substrates to be coated,
ii) coating the platelet-shaped transparent substrates with at least layers A to C and optionally layer D as claimed in claim 1.

**12.** Use of the multi-layeredpearlescent pigments having a silver interference colour as claimed in one of claims 1 to 10 in cosmetic formulations, plastics, films, textiles, ceramic material, glasses and coating compositions such as paints, printing inks, inks, varnishes or powder coatings.

**13.** Preparation containing the multi-layered pearlescent pigments as claimed in one of claims 1 to 10.

**14.** Article coated with the multi-layered pearlescent pigments as claimed in one of claims 1 to 10.

**Revendications**

**1.** Pigments nacrés multicouche dotés d'une teinte de polarisation argentée se basant sur des substrats transparents en forme de lamelles ayant un revêtement à effet optique,
**caractérisés**
**en ce que** le revêtement à effet optique comprend au moins :

a) une couche à haute réfraction A ayant un indice de réfraction $n \geq 1,8$,
b) une couche à basse réfraction B ayant un indice de réfraction $n < 1,8$,
c) une couche à haute réfraction C ayant un indice de réfraction $n \geq 1,8$, et
d) facultativement, une couche de protection extérieure D,

et **en ce que** les pigments nacrés multicouche présentent les nombres caractéristiques $D_{10}$, $D_{50}$, $D_{90}$ à partir de la répartition de fréquence cumulée de la fonction de distribution granulométrique mise à la moyenne par rapport au volume avec un écart $\Delta D$ de 0,7 à 1,4, étant entendu que l'écart $\Delta D$ est calculé selon la formule $\Delta D = (D_{90} - D_{10})/D_{50}$.

**2.** Pigments nacrés multicouche dotés d'une teinte de polarisation argentée selon la revendication 1,
**caractérisés**
**en ce que** la couche A et/ou la couche C sont absorbantes ou non absorbantes, de façon sélective et/ou non sélective, indépendamment l'une de l'autre.

**3.** Pigments nacrés multicouche dotés d'une teinte de polarisation argentée selon l'une des revendications précédentes,
**caractérisés**
**en ce que** la couche B n'est pas absorbante.

**4.** Pigments nacrés multicouche dotés d'une teinte de polarisation argentée selon l'une des revendications précédentes,
**caractérisés**
**en ce que** les pigments nacrés multicouche présentent un écart $\Delta D$ de 0,7 à 1,3.

**5.** Pigments nacrés multicouche dotés d'une teinte de polarisation argentée selon l'une des revendications précédentes,
**caractérisés**
**en ce que** l'épaisseur de la couche optique de la couche A et de la couche C se situe pour chacune dans une marge de 50 à 200 nm.

**6.** Pigments nacrés multicouche dotés d'une teinte de polarisation argentée selon l'une des revendications précédentes,
**caractérisés**
**en ce que** l'épaisseur de la couche optique de la couche B se situe dans une marge de 30 à 150 nm.

**7.** Pigments nacrés multicouche dotés d'une teinte de polarisation argentée selon l'une des revendications précédentes,
**caractérisés**

**en ce que** la couche A et/ou la couche C contiennent du dioxyde de titane et/ou de l'oxyde de fer.

**8.** Pigments nacrés multicouche dotés d'une teinte de polarisation argentée selon l'une des revendications précédentes,
**caractérisés**
**en ce que** la couche B contient du dioxyde de silicium.

**9.** Pigments nacrés multicouche dotés d'une teinte de polarisation argentée selon l'une des revendications précédentes,
**caractérisés**
**en ce que** les substrats transparents en forme de lamelles des pigments nacrés multicouche dotés d'une teinte de polarisation argentée sont choisis parmi le groupe constitué du mica naturel, du mica synthétique, du flocon de verre, des lamelles de $SiO_2$, des lamelles de $Al_2O_3$ et des mélanges de ces composants.

**10.** Pigments nacrés multicouche dotés d'une teinte de polarisation argentée selon l'une des revendications précédentes,
**caractérisés**
**en ce que** les pigments nacrés multicouche présentent une valeur chromatique $C^*_{15} \leq 20$.

**11.** Procédé de production des pigments nacrés multicouche dotés d'une teinte de polarisation argentée selon l'une des revendications précédentes, **caractérisé**
**en ce qu'**il comprend les étapes suivantes :

i) criblage granulométrique des substrats transparents en forme de lamelles devant être recouverts,
ii) recouvrement des substrats transparents en forme de lamelles avec au moins les couches A à C mentionnées dans la revendication 1, ainsi que facultativement la couche D.

**12.** Utilisation des pigments nacrés multicouche dotés d'une teinte de polarisation argentée selon l'une des revendications 1 à 10 dans des formules cosmétiques, des plastiques, des feuilles, des textiles, des matériaux céramiques, des verres et des composés de recouvrement tels que des peintures, des encres d'impression, des encres, des vernis ou des peintures en poudre.

**13.** Préparation qui contient les pigments nacrés multicouche selon l'une des revendications 1 à 10.

**14.** Objet qui est recouvert avec des pigments nacrés multicouche selon l'une des revendications 1 à 10.

Abbildung 1:

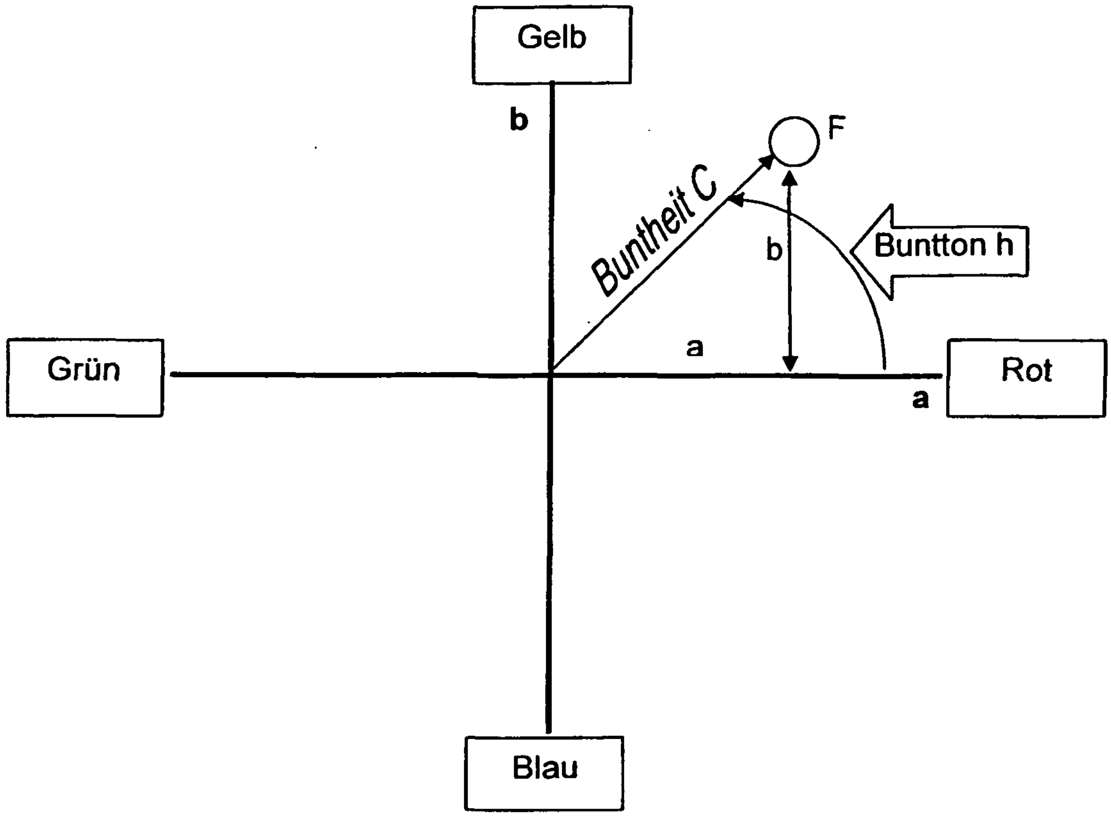

Abbildung 2:

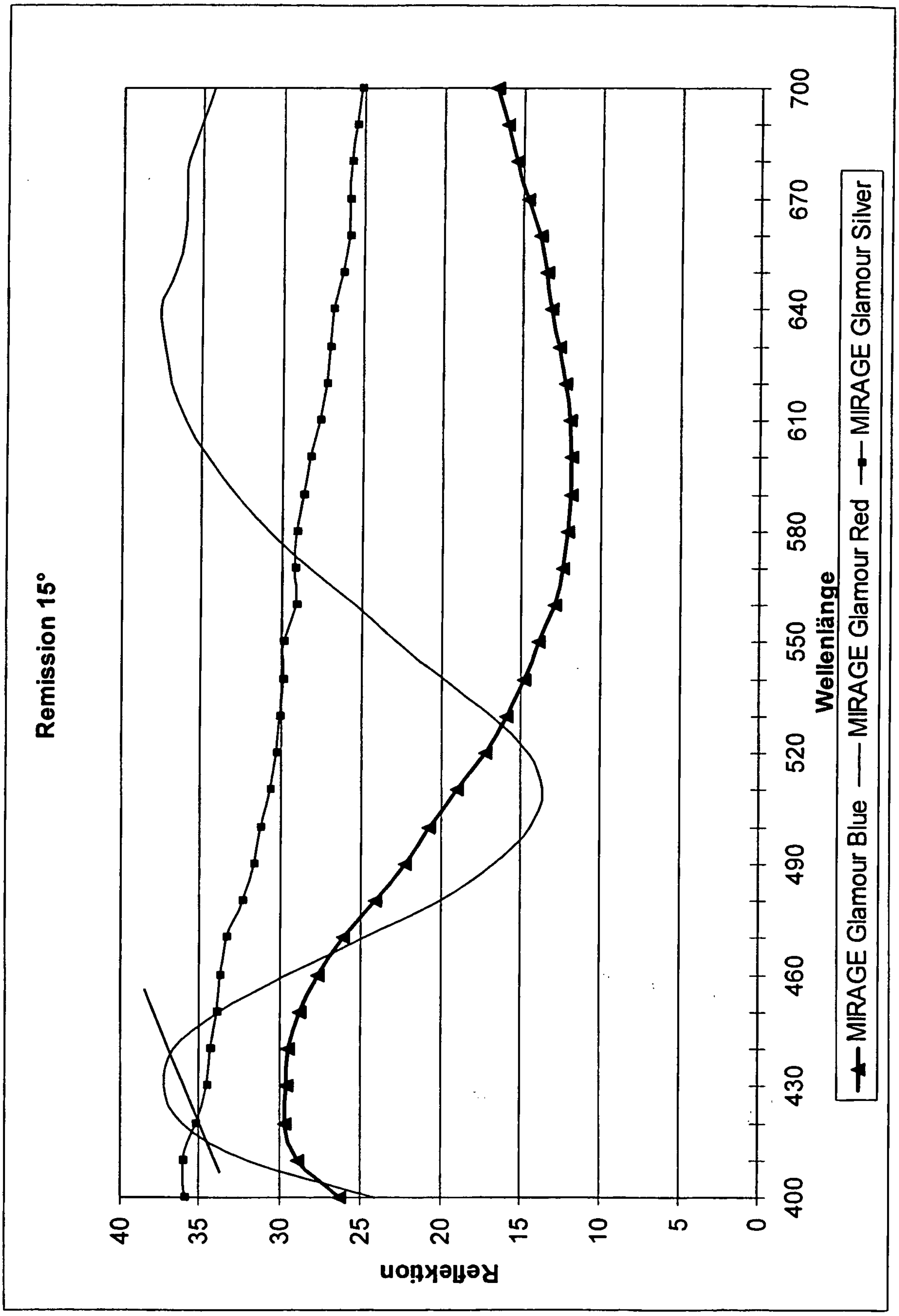

Remission 15°
Reflektion
Wellenlänge
0
5
10
15
20
25
30
35
40
400
430
460
490
520
550
580
610
640
670
700
MIRAGE Glamour Blue
MIRAGE Glamour Red
MIRAGE Glamour Silver

Abbildung 3: Beeinflussung der Laserbeugung durch Partikeleigenschaften

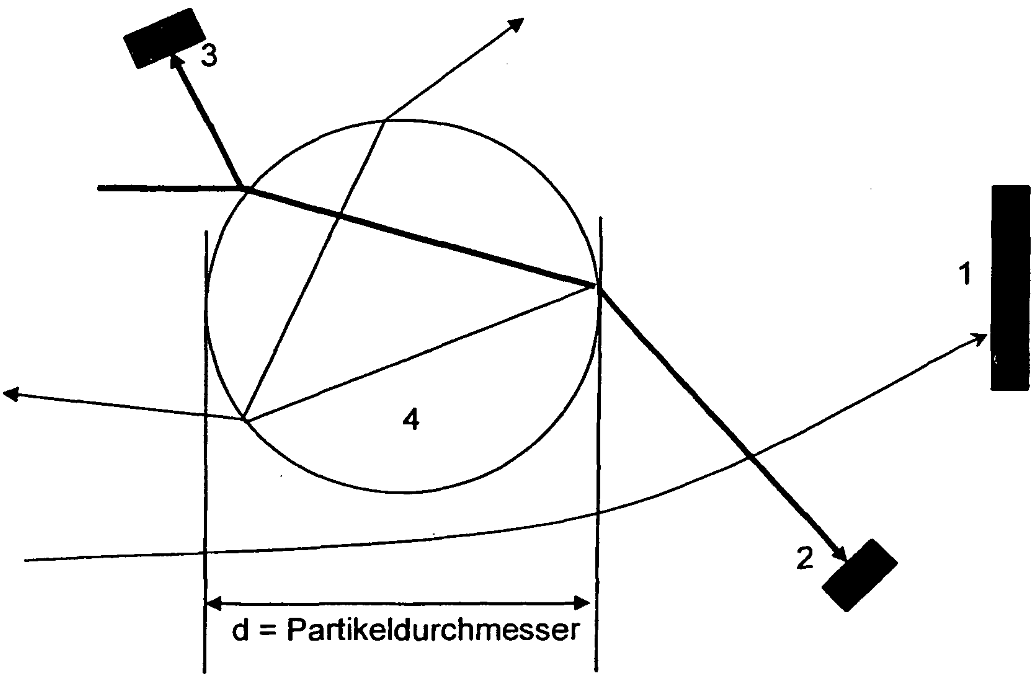


Partikeleigenschaften:

d: Durchmesser

1:Beugung

2: Brechung

3 Reflexion

4: Absorption

Abbildung 4:

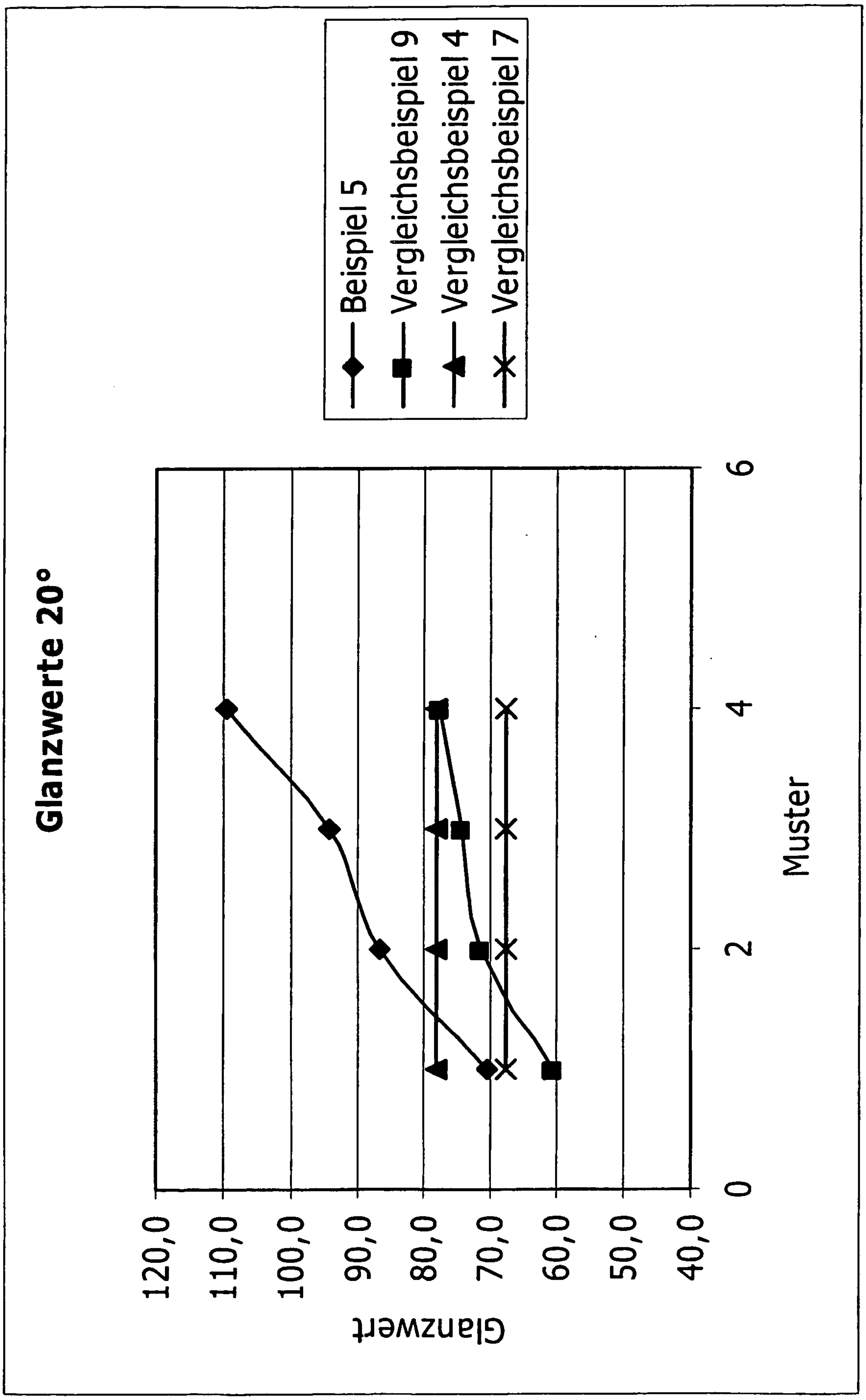
Glanzwerte 20°
Glanzwert
120,0
110,0
100,0
90,0
80,0
70,0
60,0
50,0
40,0
0
2
4
6
Muster
Beispiel 5
Vergleichsbeispiel 9
Vergleichsbeispiel 4
Vergleichsbeispiel 7

Abbildung 5:

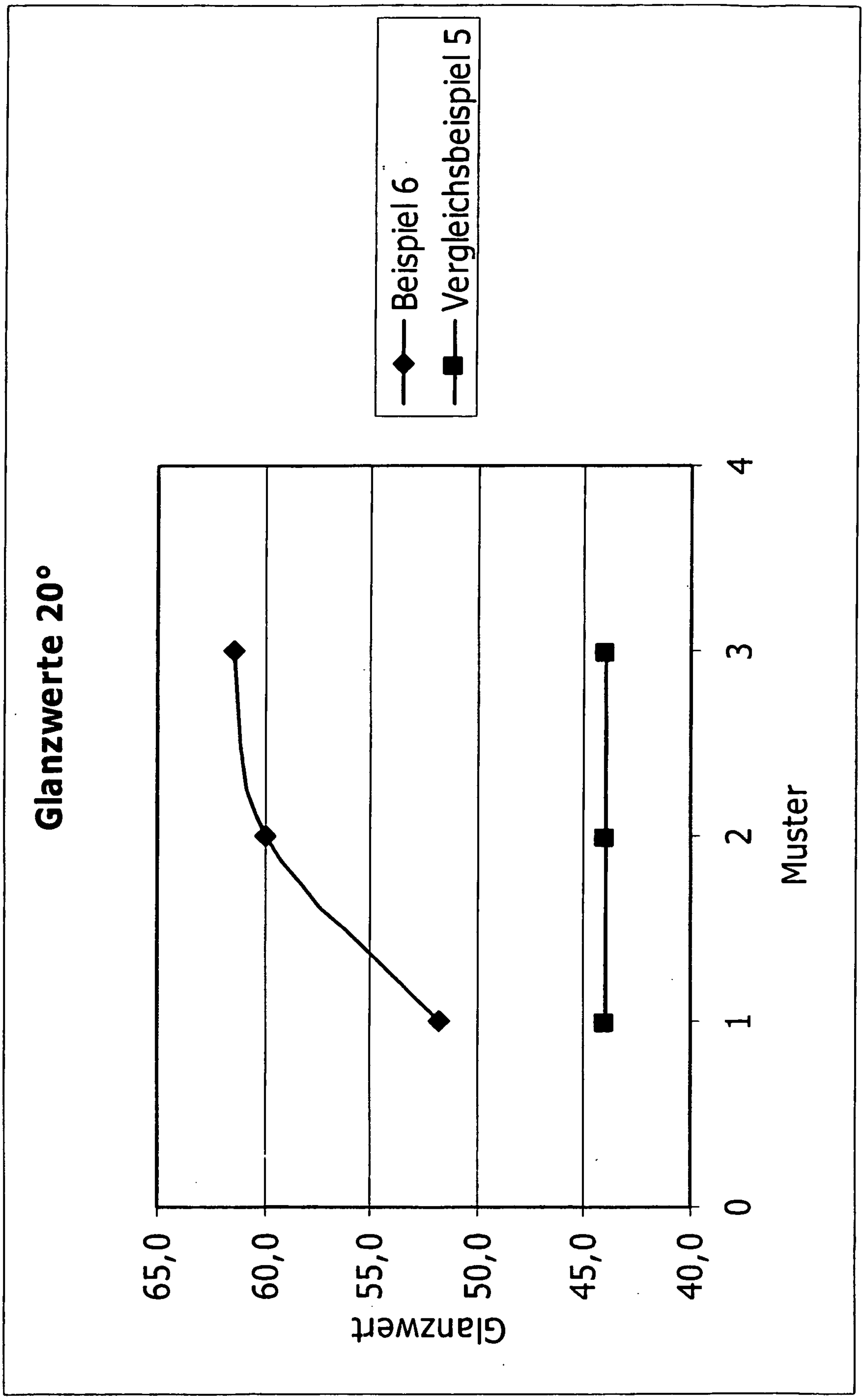
Glanzwerte 20°
Glanzwert
65,0
60,0
55,0
50,0
45,0
40,0
0
1
2
3
4
Muster
Beispiel 6
Vergleichsbeispiel 5

Abbildung 6:

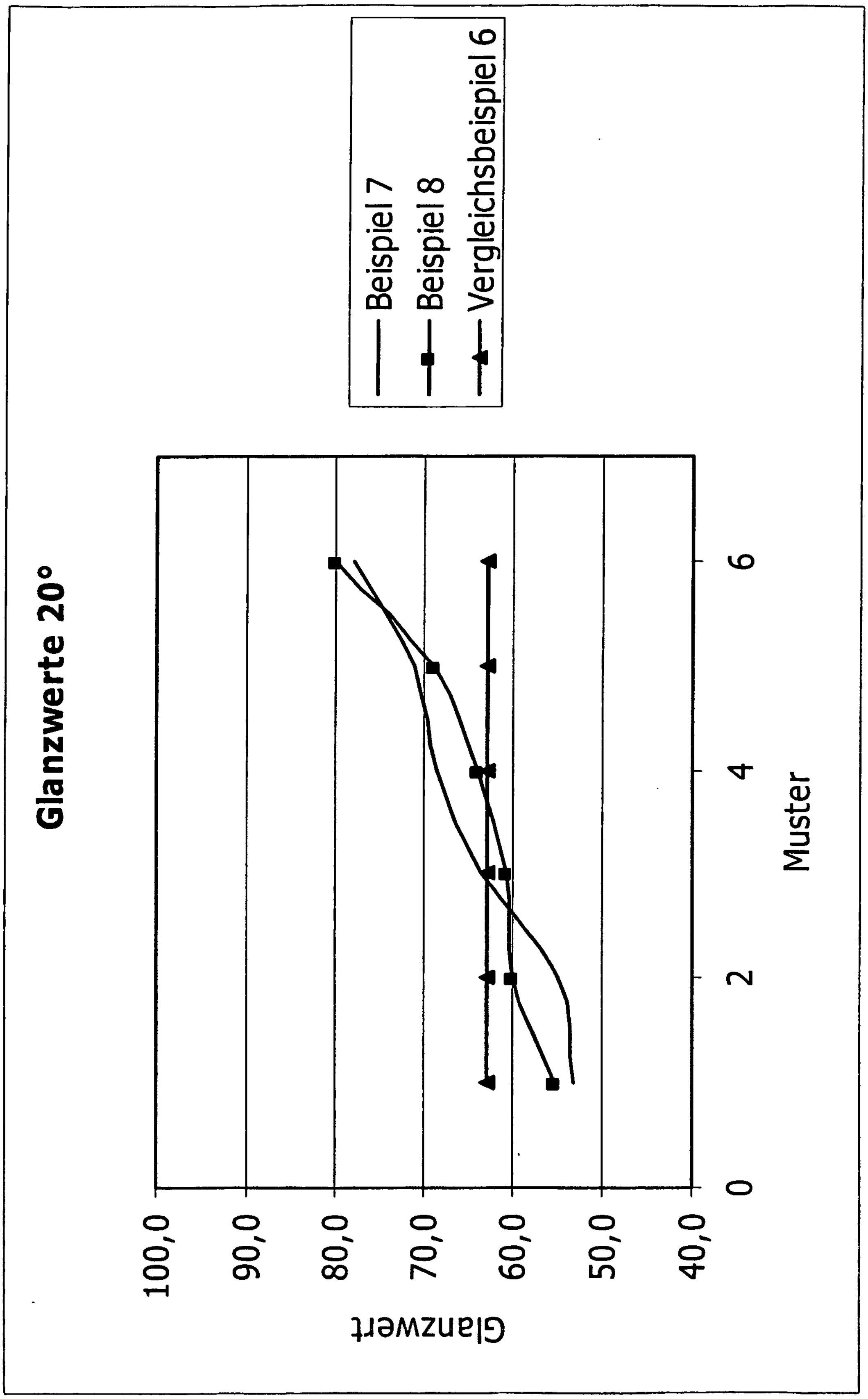
Glanzwerte 20°
Glanzwert
100,0
90,0
80,0
70,0
60,0
50,0
40,0
0
2
4
6
Muster
Beispiel 7
Beispiel 8
Vergleichsbeispiel 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1213330 A1
- EP 0753545 B2
- WO 2004067645 A2
- WO 2006088759 A1
- US 20060042509 A
- WO 2009103322 A1
- EP 0948572 B1
- JP 07246366 B
- EP 1025168 B1
- WO 2003006558 A2
- WO 2004055119 A1
- WO 2002090448 A2
- WO 2006110359 A2
- EP 0289240 A1
- WO 2004056716 A1
- WO 2005063637 A1
- EP 1980594 B1
- WO 2006021386 A1